# EUROPEAN PATENT SPECIFICATION

(11) **EP 1 003 766 B1**
(45) Date of publication and mention of the grant of the patent: **29.04.2009**
(21) Application number: 98922352.4
(22) Date of filing: 15.05.1998
(51) Int. Cl.: C07H 21/04, C07K 14/705, G01N 33/53, C12N 15/63, C12N 15/12, C07K 16/18, C07K 16/28, C12Q 1/68, G01N 33/50

(54) **TUMOR SURFACE PROTEIN AND ITS COMPLEMENTARY SURFACE RECEPTOR ON NAIVE NATURAL KILLER CELLS**
TUMOR-OBERFLÄCHENPROTEIN UND SEIN ENTSPRECHENDER OBERFLÄCHENREZEPTOR AUF NATÜRLICHEN KILLERZELLEn
PROTEINE DE SURFACE DE CELLULE TUMORALE ET SON RECEPTEUR DE SURFACE COMPLEMENTAIRE SUR DES LYMPHOCYTES TUEURS NATURELS NAIFS

(30) Priority: 15.05.1997 US 46553 P
(43) Date of publication of application: 31.05.2000
(73) Proprietor: THE RESEARCH FOUNDATION OF STATE UNIVERSITY OF NEW YORK, Albany, New York 12201-0009 (US)
(72) Inventor: NORIN, Allen, J., Pomona, NY 10970 (US)
(74) Representative: Prins, Adrianus Willem
(86) International application number: PCT/US1998/010021
(87) International publication number: WO 1998/051701

(56) References cited:
- DAS BALLABH ET AL: "Preferential interaction of a novel tumor surface protein (p38.5) with naive natural killer cells." JOURNAL OF EXPERIMENTAL MEDICINE, vol. 185, no. 10, 1997, pages 1735-1742, XP009005782 ISSN: 0022-1007
- TAO S -Z ET AL: "Molecular cloning of p38.5-K562 tumor membrane protein: A specific ligand for human natural killer (NK) cells." PROCEEDINGS OF THE AMERICAN ASSOCIATION FOR CANCER RESEARCH ANNUAL, vol. 37, 1996, page 458 XP001145448 87th Annual Meeting of the American Association for Cancer Research;Washington, D.C., USA; April 20-24, 1996, 1996 ISSN: 0197-016X
- VITALE MASSIMO ET AL: "Physical and functional independency of p70 and p58 natural killer (NK) cell receptors for HLA class I: Their role in the definition of different groups of alloreactive NK cell clones." PROCEEDINGS OF THE NATIONAL ACADEMY OF SCIENCES OF THE UNITED STATES, vol. 93, no. 4, 1996, pages 1453-1457, XP002232312 1996 ISSN: 0027-8424
- DATABASE EMBL [Online] mp75g11.r1 Soares 2NbMt Mus musculus cDNA clone, 21 November 1996 (1996-11-21) MARRA ET AL: "The WashU HHMI EST project" retrieved from EBI Database accession no. aa120682 XP002231496
- DATABASE EMBL [Online] yx44b11.r1 Soares melanocyte 2NbHM homo s. cDNA , 31 December 1995 (1995-12-31) HILLIER ET AL: "The WashU-Merck EST Project" retrieved from EBI Database accession no. n27595 XP002231497
- DATABASE EMBL [Online] yx44b11.s1 Soares melanocyte 2NbHM homo s cDNA, 31 December 1995 (1995-12-31) HILLIER ET AL: "The WashU-Merck EST Project" retrieved from EBI Database accession no. N20321 XP002232394
- OBEXER G ET AL: "TARGET EFFECTOR CELL INTERACTIONS IN THE HUMAN NATURAL KILLER SYSTEM ISOLATION OF TARGET STRUCTURES" IMMUNOBIOLOGY, vol. 165, no. 1, 1983, pages 15-26, XP009005783 ISSN: 0171-2985
- DAS et al., "Identification of a Novel NK Cell Specific Receptor-Ligand System", PROC. AM. ASSOC. CANCER RES., page 472, Abstract 2811, XP002912729
- FASEB J., Volume 10, Number 6, issued 1996, TAO et al., "Molecular Cloning of 38.5 K562 Tumor Membrane Protein: A Specific Ligand for Human Natural Killer (NK) Cells", page A1346, Abstract 2002, XP002912730
- NORIN et al., "A 38.5 KD Tumor Protein is a Unique Recognition Structure for Naive Human NK Cells", J. CELL. BIOCHEM. SUPPL., 1993, page 123, Abstract NZ418, XP002912731

### SEQUENCE LISTING

<110> The Research Foundation of State University of New York
<120> Tumor Surface Protein and Its Complementary Surface Receptor on Naive Natural Killer Cells
<130> P51688EP00
<140> 98922352.4
   <141> 1999-12-02
<150> US 60/046,553
   <151> 1997-05-15
<160> 5
<170> Patent In Ver. 2.1
<210> 1
   <211> 730
   <212> DNA
   <213> Homo sapiens
<220>
   <223> /note="P38.5 cDNA wherein Y can be C or T, K can be G or T and R can be G or A"
<400> 1
   gagggagtgg agaaactgct gttcaaacaa ggyctttgca ggggatggga ggagtgggtc 60
   aagctcacag tcaacaaagg gttgagtaac cattttcagg tcaaccacac agtagccctc 120
   agcacaatcg gggagtccaa ctaccacttc ggggtcacat atgtggggac aaagcagctg 180
   agtcccacag aggcgttccc tgtactggtg ggtgacatgg acaacagtgg cagtctcaac 240
   gctcaggtca ttcaccagct gggccccggt ctcaggtcca agatggccat ccagacccag 300
   cagtcgaagt ttgtgaactg gcaggtggac ggggagtatc ggggctctga cttcacagca 360
   gccgtcaccc tggggaaccc agacgtcctc gtgggttcag gaatcctcgt akcccrctac 420
   ctccagagca tcacgccttg cctggccctg ggtggagagc tggtctacca ccggcggcct 480
   ggagaggagg gcactgtcat gtctctagct gggaaataca cattgaacaa ctggttggca 540
   acggtaacgt tgggccaggc gggcatgcac gcaacatact accacaaagc cagtgaccag 600
   ctgcaggtgg gtgtggagtt tgaggccagc acaaggatgc aggacaccag cgtctccttc 660
   gggtaccagc tggacctgcc caaggccaac ctctttttca aaggctctgt ggatagcaac 720
   tggatcgtgg                                                        730
<210> 2
   <211> 243
   <212> PRT
   <213> Homo sapiens
<220>
   <223> /note="P38.5 Werein position 138 can be Ala or Ser and position 139 can be Arg or His"
<400> 2
   Glu Gly Val Glu Lys Leu Leu Phe Lys Gln Gly Leu Cys Arg Gly Trp
    1               5                  10                  15
   Glu Glu Trp Val Lys Leu Thr Val Asn Lys Gly Leu Ser Asn His Phe
               20                  25                  30
   Gln Val Asn His Thr Val Ala Leu Ser Thr Ile Gly Glu Ser Asn Tyr
           35                  40                  45
   His Phe Gly Val Thr Tyr Val Gly Thr Lys Gln Leu Ser Pro Thr Glu
       50                  55                  60
   Ala Phe Pro Val Leu Val Gly Asp Met Asp Asn Ser Gly Ser Leu Asn
   65                  70                  75                  80
   Ala Gln Val Ile His Gln Leu Gly Pro Gly Leu Arg Ser Lys Met Ala
                   85                  90                  95
   Ile Gln Thr Gln Gln Ser Lys Phe Val Asn Trp Gln Val Asp Gly Glu
               100                 105                 110
   Tyr Arg Gly Ser Asp Phe Thr Ala Ala Val Thr Leu Gly Asn Pro Asp
           115                 120                 125
   Val Leu Val Gly Ser Gly Ile Leu Val Ala Arg Tyr Leu Gln Ser Ile
       130                 135                 140
   Thr Pro Cys Leu Ala Leu Gly Gly Glu Leu Val Tyr His Arg Arg Pro
   145                 150                 155                 160
   Gly Glu Glu Gly Thr Val Met Ser Leu Ala Gly Lys Tyr Thr Leu Asn
                   165                 170                 175
   Asn Trp Leu Ala Thr Val Thr Leu Gly Gln Ala Gly Met His Ala Thr
               180                 185                 190
   Tyr Tyr His Lys Ala Ser Asp Gln Leu Gln Val Gly Val Glu Phe Glu
           195                 200                 205
   Ala Ser Thr Arg Met Gln Asp Thr Ser Val Ser Phe Gly Tyr Gln Leu
       210                 215                 220
   Asp Leu Pro Lys Ala Asn Leu Phe Phe Lys Gly Ser Val Asp Ser Asn
   225                 230                 235                 240
   Trp Ile Val
<210> 3
   <211> 11
   <212> PRT
   <213> Homo sapiens
<220>
   <221> PEPTIDE
   <222> (1)..(11)
<400> 3
   Phe Val Asn Trp Gln Val Asp Gly Glu Tyr Arg
     1               5                   10
<210> 4
   <211> 1315
   <212> DNA
   <213> Homo sapiens
<220>
   <223> /note="P70 cDNA wherein K can be T or G and R can be A or G"
<400> 4
   ttcggcacga gccattttca ggtcaaccac acagtagccc tcagcacaat cggggagtcc 60
   aactaccact tcggggtcac atatgtgggg acaaagcagc tgagtcccac agaggcgttc 120
   cctgtactgg tgggtgacat ggacaacagt ggcagtctca acgctcaggt cattcaccag 180
   ctgggccccg gtctcaggtc caagatggcc atccagaccc agcagtcgaa gtttgtgaac 240
   tggcaggtgg acggggagta tcggggctct gacttcacag cagccgtcac cctggggaac 300
   ccagacgtcc tcgtgggttc aggaatcctc gtakcccrct acctccagag catcacgcct 360
   tgcctggccc tgggtggaga gctggtctac caccggcggc ctggagagga gggcactgtc 420
   atgtctctag ctgggaaata cacattgaac aactggttgg caacggtaac gttgggccag 480
   gcgggcatgc acgcaacata ctaccacaaa gccagtgacc agctgcaggt gggcgtggag 540
   tttgaggcca gcacaaggat gcaggacacc agcgtctcct tcgggtacca gctggacctg 600
   cccaaggcca acctcctctt caaaggctct gtggatagca actggatcgt gggtgccacg 660
   ctggagaaga agctcccacc cctgcccctg acactggccc ttggggcctt cctgaatcac 720
   cgcaagaaca agtttcagtg tggctttggc ctcaccatcg gctgagccct cctggccccc 780
   gccttccacg cccttccgat tccacctcca cctccacctc cccctgccac agaggggaga 840
   cctgagcccc cctcccttcc ctcccccctt gggggtcggg ggggacattg gaaaggaggg 900
   accccgccac cccagcagct gaggagggga ttctggaact gaatggcgct tcgggattct 960
   gagtagcagg ggcagcatgc ccagtgggcc tggggtcccg ggagggattc cagaattgag 1020
   gggcacgcag gattctgagc accaggggca gaggcggcca gacaacctca gggaggagtg 1080
   tcctggcgtc cccatcctcc aaagggcctg ggcccgcccc gagggggcag cgagaggagc 1140
   ttccccatcc ccggtcagtc caccctgccc cgtccacttt cccatctcct cggtataaat 1200
   catgtttata agttatggaa gaaccgggac attttacaga aaaaaaacaa aaaacaacaa 1260
   aaaatatacg tgggaaaaaa aaaaaaaaaa aaaaaaaaaa aaaaaaaaaa aaaaa      1315
<210> 5
   <211> 254
   <212> PRT
   <213> Homo sapiens
<220>
   <223> /note="P70 wherein position 112 can be Ser or Ala and position 113 can be His or Arg"
<400> 5
   Phe Gly Thr Ser His Phe Gln Val Asn His Thr Val Ala Leu Ser Thr
     1               5                  10                  15
   Ile Gly Glu Ser Asn Tyr His Phe Gly Val Thr Tyr Val Gly Thr Lys
                20                  25                  30
   Gln Leu Ser Pro Thr Glu Ala Phe Pro Val Leu Val Gly Asp Met Asp
            35                  40                  45
   Asn Ser Gly Ser Leu Asn Ala Gln Val Ile His Gln Leu Gly Pro Gly
        50                  55                  60
   Leu Arg Ser Lys Met Ala Ile Gln Thr Gln Gln Ser Lys Phe Val Asn
    65                  70                  75                  80
   Trp Gln Val Asp Gly Glu Tyr Arg Gly Ser Asp Phe Thr Ala Ala Val
                    85                  90                  95
   Thr Leu Gly Asn Pro Asp Val Leu Val Gly Ser Gly Ile Leu Val Ser
               100                 105                 110
   His Tyr Leu Gln Ser Ile Thr Pro Cys Leu Ala Leu Gly Gly Glu Leu
           115                 120                 125
   Val Tyr His Arg Arg Pro Gly Glu Glu Gly Thr Val Met Ser Leu Ala
       130                  135                 140
   Gly Lys Tyr Thr Leu Asn Asn Trp Leu Ala Thr Val Thr Leu Gly Gln
   145                 150                 155                 160
   Ala Gly Met His Ala Thr Tyr Tyr His Lys Ala Ser Asp Gln Leu Gln
                   165                 170                 175
   Val Gly Val Glu Phe Glu Ala Ser Thr Arg Met Gln Asp Thr Ser Val
               180                 185                 190
   Ser Phe Gly Tyr Gln Leu Asp Leu Pro Lys Ala Asn Leu Leu Phe Lys
           195                 200                 205
   Gly Ser Val Asp Ser Asn Trp Ile Val Gly Ala Thr Leu Glu Lys Lys
       210                 215                 220
   Leu Pro Pro Leu Pro Leu Thr Leu Ala Leu Gly Ala Phe Leu Asn His
   225                 230                 235                 240
   Arg Lys Asn Lys Phe Gln Cys Gly Phe Gly Leu Thr Ile Gly
                   245                 250

## Description

This application claims the benefit of U.S. Provisional Application No. 60/046,553, filed May 15, 1997.

This invention was made with government support under Grant No. RO1CA47548 sponsored by the National Institutes of Health. The government has certain rights in the invention.

### FIELD OF THE INVENTION

The present invention relates to surface receptors on tumor cells susceptible to Natural Killer (NK) cells.

### BACKGROUND OF THE INVENTION

Naive natural killer (NK) cells (CD3⁻, CD16⁺ TCR⁻), unlike cytolytic T lymphocytes (CTL) (CD3⁺, CD16⁻, TCR⁺) provide cell-mediated lytic activity against virus infected cells and certain tumors without requirement for activation. Trinchieri, G. Adv. Immunol. 47:187-376 1989; Whiteside, T.L. and R.B.Herberman Immunol. Allergy Clin. N. Amer. 10:663-704 (1990); Bancroft, G.J. Curr. Opin. Immunol. 5:503-510(1993). Such unactivated lymphocytes, also referred to as resting or naive NK cells, are capable of destroying a spectrum of tumor cells. Trinchieri, G. Adv. Immunol. 47:187-376 (1989); Whiteside T.L., in Current Protocols in Immunology, J.E. Coligan et al. eds., Supplement 17, Unit 7-18, John Wiley & Sons, New York (1996). Upon activation with lymphokines such as Il-2, NK cells acquire broad antitumor lytic activity (lymphokine activated killer cells i.e LAK cells). The mechanism(s) by which naive NK cells recognize their target cells is not completely understood. Interaction of cellular adhesion molecules and recognition of specific target structure(s) have been proposed as critical initial events in the cell mediated lytic process. Trinchieri, G. Adv. Immunol. 47:187-376 (1989); Storkus, W.J. and J.R. Dawson Critical Rev. Immunol. 10:393-416 (1989). It is well known, for example, that the initiation of target cell lysis by CTL is due to interaction of the major histocompatibility complex (MHC) class I molecules (plus bound peptide) with the T cell receptor (TCR). Davis, M.M. and P.J. Bjorkman. Nature (Lond.) 334:395-401 (1988); Townsend, A. and H. Bodmer Ann. Rev. Immunol. 7:601-624 (1989). Analogous molecular structures that initiate the lytic process between NK cells and tumor cells have not been defined. Although MHC molecules may serve a regulatory function for NK cells (Gumperz, J.E. and P. Perham Nature (Lond.) 378:245-248 (1995); Yokoyama, W.M. Proc. Natl. Acad Sci. (USA) 92:3081-3085 (1995); Lanier, L.L. and J.H. Phillips Immunol. Today 17:86-91 (1996); Moretta, A., S. Sivori, M. Vitale. D. Pende, L. Morelli, R. Augugliaro, C. Bottino, and L. Moretta J.Exp. Med. 182:875-884 (1995)), it is clear that their presence on the surface of tumor cells is not required for cytolysis since many NK susceptible cell lines do not express MHC gene products. Moretta, A., S. Sivori, M. Vitale, D. Pende, L. Morelli, R. Augugliaro, C. Bottino, and L. Moretta J.Exp. Med 182:875-884 (1995); Litwin, V., J. Gumperz, P. Parham. J.H. Phillips and L.L. Lanier J. Exp. Med. 178:1321-1336 (1993).

Previous attempts during the past two decades to identify recognition structures exclusive to NK cell-tumor interaction have been unsuccessful (Gumperz, J.E. and P. Perham Nature (Lond.) 378:245-248 (1995) though important components on both NK cells and on tumor cells that contribute to cellular adhesion and regulation of the cytolytic process have been revealed. Storkus, W.J. and J.R. Dawson Critical Rev. Immunol. 10:393-416 (1991); Gumperz, J.E. and P. Perham Nature (Lond.) 378:245-248 (1995); Yokoyama, W.M. Proc. Natl. Acad. Sci. (USA) 92:3081-3085 (1995); Lanier, L.L. and J.H. Phillips Immunol. Today 17:86-91 (1995); Moretta, A., S. Sivori, M. Vitale, D. Pende, L. Morelli, R. Augugliaro, C. Bottino, and L. Moretta J.Exp. Med. 182:875-884 (1995); Litwin, V., J. Gumperz, P. Parham, J.H. Phillips and L.L. Lanier J. Exp. Med. 178:1321-1336 (1993). These receptor- ligand interactions, however, are not unique to NK cells since they also occur between T lymphocytes and their respective target cells (Lanier, L.L. and J.H. Phillips Immunol. Today 17:86-91 (1996).

The limited number of NK cells available for biochemical studies (<5% in peripheral blood) has undoubtedly contributed to the difficulty in analysis of NK cell specific receptor-ligand interactions. Many investigators have addressed this issue by the expansion of freshly isolated NK cells in vitro utilizing a variety of culture systems usually employing growth factors and feeder cells. Moretta, A., S. Sivori, M. Vitale, D. Pende, L. Morelli, R. Augugliaro, C. Bottino, and L. Moretta J.Exp. Med. 182:875-884 (1995); Litwin, V., J. Gumperz, P. Parham, J.H. Phillips and L.L. Lanier J Exp. Med 178:1321-1336 (1993); Windebank, K.P., R.T. Abraham, G. Powis, R.A. Olsen, T.J. Barna and P.J. Leibson J. Immunol. 141:3951-3957 (1988). Such techniques have provided sufficient numbers of cells for immunologic and biochemical studies but at the expense of altering the phenotype of the freshly isolated NK cells from a naive state to an activated one, as demonstrated by induction of the Lag 3 protein (Triebel, F., S. Jitsukawa, F. Baixeras, S. Roman-Roman, C. Genevee, E. Viegas-Pequignot and T. Hercend J. Exp. Med 171:1393-1405 (1990). Thus, the surface proteins responsible for NK cell specific receptor-ligand interactions still remain largely unknown.

Accordingly, there is a need in the art to ascertain the surface proteins responsible for NK cell specific receptor-ligand interactions. Moreover, there is a need in the art for diagnostic methods that accurately establish the presence of NK susceptible cancers, and for therapeutic methods to treat mammals suffering from these cancers.

### SUMMARY OF THE INVENTION

It has now been discovered that these and other objectives can be achieved by the present invention, which provides an isolated and purified cell surface protein p38.5 having a molecular weight of about 38.5 kD which binds Natural Killer (NK) cells and which is characteristically expressed by tumor cells susceptible to cell-mediated lysis by naive Natural Killer (NK) cells. The protein p38.5 binds preferentially to NK cells in the presence of other types of lymphocytes.

The protein p38.5 binds to p70, a protein which is characteristically expressed on the surface ofNK cells. Protein p38.5 can be from a mammal. The mammal can be a human.

Protein p38.5 comprises an amino acid sequence defined by SEQ ID NO:2, a unique fragment thereof, or a homolog thereof.

Also described is a protein comprising an amino acid sequence defined by SEQ ID NO:2 or a unique fragment thereof. Also described is the amino acid sequence defined by SEQ ID NO:3.

In another embodiment the invention is a nucleic acid encoding a cell surface protein p38.5 having a molecular weight of about 38.5 kD which binds Natural Killer (NK) cells and which is characteristically expressed by tumor cells susceptible to cell-mediated lysis by naive Natural Killer (NK) cells, wherein the protein comprises the amino acid sequence defined by SEQ ID NO: 3. This nucleic acid can be DNA. RNA, or cDNA.

Also described is a nucleic acid which encodes a polypeptide having an amino acid sequence selected from the group consisting of SEQ ID NO:2, SEQ ID NO:3, and unique fragments thereof. This nucleic acid can also comprise the nucleotide sequence defined by SEQ ID NO:1 or a unique fragment thereof. The nucleic acid can also be a vector comprising the nucleotide sequence. This vector can be, for example, a plasmid, a phage, or a cosmid.

Also described is a nucleic acid which is a vector having ATCC Deposit number 98422.

Also described is an oligonucleotide which hybridizes specifically with a nucleic acid comprising a nucleotide sequence defined by SEQ ID NO: 1. This oligonucleotide can further comprise a detectable label moiety.

In another embodiment, the invention is an antibody having specific binding affinity for an epitope of a cell surface protein p38.5 having a molecular weight of about 38.5 kD which binds Natural Killer (NK) cells and which is characteristically expressed by tumor cells susceptible to cell-mediated lysis by naive Natural Killer (NK) cells, wherein the protein comprises the amino acid sequence defined by SEQ ID NO: 3. The antibody of the invention can also be selected from the group consisting of monoclonal antibodies, polyclonal antibodies, chimeric antibodies, humanized antibodies, Fv fragments, and Fab'₂ fragments.

The antibody may specifically bind to an epitope characteristic of the amino acid sequence defined by SEQ ID NO:2 or a unique fragment thereof. In a preferred embodiment, the antibody of the invention specifically binds to an epitope characteristic of the amino acid sequence defined by SEQ ID NO:3.

Also described is an isolated and purified cell surface protein p70 having a molecular weight of about 70 kD which is characteristically expressed by naive NK cells and which preferentially binds to tumor cells that are susceptible to NK cell-mediated lysis. This protein binds to a cell surface protein p38.5 characteristically expressed by NK-susceptible tumor cells.

Cell surface protein p70 can be from a mammal. The mammal can be a human.

Also described is a nucleic acid encoding a cell surface protein p70 having a molecular weight of about 70 kD which is characteristically expressed by Natural Killer (NK) cells and which binds to tumor cells susceptible to cell-mediated lysis by naive (NK) cells. The nucleic acid can be DNA, RNA, or CDNA.

Also described is a nucleic acid which encodes a polypeptide having an amino acid sequence selected from the group consisting of SEQ ID NO:5 and unique fragments thereof. Also described is a nucleic acid comprising the nucleotide sequence

Also described is a nucleic acid comprising the nucleotide sequence defined by SEQ ID NO:4 or a unique fragment thereof. This nucleic acid can be a vector comprising this nucleotide sequence. The vector can be, for example, a plasmid, a phage, or a cosmid. This nucleic acid can also be a vector having ATCC deposit number 98424.

Also described is an oligonucleotide which hybridizes specifically with a nucleic acid comprising a nucleotide sequence defined by SEQ ID NO:4. This ologonucleotide further comprise a detectable label moiety.

Also described is an antibody that specifically binds to an epitope of a Natural Killer (NK) cell surface protein p70, wherein said protein has a molecular weight of about 70 kD which binds tumor cells susceptible to cell-mediated lysis by naive Natural Killer (NK) cells. This antibody can be selected from the group consisting of monoclonal antibodies, polyclonal antibodies, chimeric antibodies, humanized antibodies, Fv fragments, and Fab'₂ fragments. A preferred antibody specifically binds to an epitope characteristic of the amino acid sequence defined by SEQ ID NO:5 or a unique fragment thereof.

Also described is a bacteriophage containing cDNA inserts encoding the entire naive human. NK cell library and which have the ATCC accession number 98423. Preferably, the library is enriched for unique NK cell cDNA.

In yet another embodiment, the invention is an in vitro diagnostic method for identifying tumor cells that are susceptible to natural killer (NK) cell-mediated lysis, comprising:
(a) providing a sample suspected of containing tumor cells obtained from an animal subject; and
(b) detecting expression of the p38.5 cell surface protein as defined herein by the tumor cells;
wherein detection of a measurable amount of the protein is indicative of susceptibility of said tumor cells being susceptible to NK cell-mediated lysis.

In the diagnostic method for identifying tumor cells that are susceptible to natural killer (NK) cell-mediated lysis, the detecting can comprise:
(i) contacting the tumor cells with an antibody having specific binding affinity for the p38.5 protein; and
(ii) measuring binding of the antibody to the tumor cells,
   wherein measurable binding of the antibody to the tumor cells is indicative of susceptibility of said tumor cells being susceptible to NK cell-mediated lysis.

The antibody in this method, in a preferred embodiment, binds specifically to a protein comprising the amino acid sequence defined by SEQ ID NO:2 or a unique fragment thereof. In another preferred embodiment, the antibody binds specifically to a protein comprising the amino acid sequence defined by SEQ ID N0:3.

In yet another preferred embodiment of the diagnostic method for identifying tumor cells that are susceptible to natural killer (NK) cell-mediated lysis, the detecting comprises:
(i) contacting said sample under hybridization conditions with an oligonucleotide probe that hybridizes specifically with a nucleic acid encoding the p38.5 protein; and
(ii) measuring hybridization of said oligonucleotide probe to said sample, wherein measurable hybridization of the oligonucleotide probe to the sample is indicative of the presence of tumor cells susceptible to NK cell-mediated lysis.

The oligonucleotide probe can, in a preferred embodiment, bind to an mRNA encoding the p38.5 protein. In a preferred embodiment the measuring comprises measuring amplification of nucleic acid encoding the p38.5 protein mediated through the oligonucleotide probe.

The hybridizing, in a preferred embodiment, comprises hybridizing the oligonucleotide probe with the nucleic acid defined by SEQ ID NO: 1 or a unique fragment thereof.

Detecting can also also be by hybridizing with a nucleic acid probe to mRNA expressing p70 or a unique fragment of p70.

Also described is a method for determining the number of natural killer (NK) cells in a biological sample, comprising:
(a) contacting the sample containing NK cells with an antibody comprising a detectable label moiety and having specific binding affinity for p70 protein, under conditions permissive for binding of the antibody to p70 protein expressed by NK cells in said sample; and
(b) counting the number of cells bound by said antibody, thereby permitting determination the number of natural killer cells in the sample.

The detectable label moiety, in preferred embodiments can be selected from the group consisting of fluorescent compounds, colored compounds, enzymes, members of specific binding pairs, polymer beads, and magnetic beads.

These and other advantages of the invention will be appreciated from the detailed description and examples which are set forth herein. The detailed description and examples enhance the understanding of the invention, but are not intended to limit the scope of the invention.

### BRIEF DESCRIPTION OF THE DRAWINGS

Figure 1 is a photograph of Western blots demonstrating the Binding of K562 membrane proteins to subsets of Human peripheral blood lymphocytes (HPBL).
Figure 2 is a photograph of Western blots demonstrating the binding of purified p38.5 to HPBL.
Figure 3A to Figure 3F are plots from flow cytometry analysis of anti-p38.5 - treated NK-susceptible and NK-resistant tumor cell lines.
Figure 3G is a photograph of Western blots of anti-p38.5 immunoprecipitates from surface labeled cells.
Figure 4 is a bar graph illustrating the relationship between cell surface expression of p38.5 on normal and variant tumor cell lines and susceptibility of tumors to NK cell mediated lysis.
Figure 5 is a bar graph illustrating the effect of purified K562 membrane proteins on naive NK cell-mediated cytotoxicity.
Figure 6 is a photograph of Western blots identifying the direct interaction of p38.5-binding naive NK cell membrane protein.
Figure 7 is a photograph of Western blots of the p38.5 ligand bound to the 70 kD NK cell protein.
Figure 8 is a photograph of Western blots illustrating the plasma membrane localization of the 70 kD NK cell tumor binding protein.

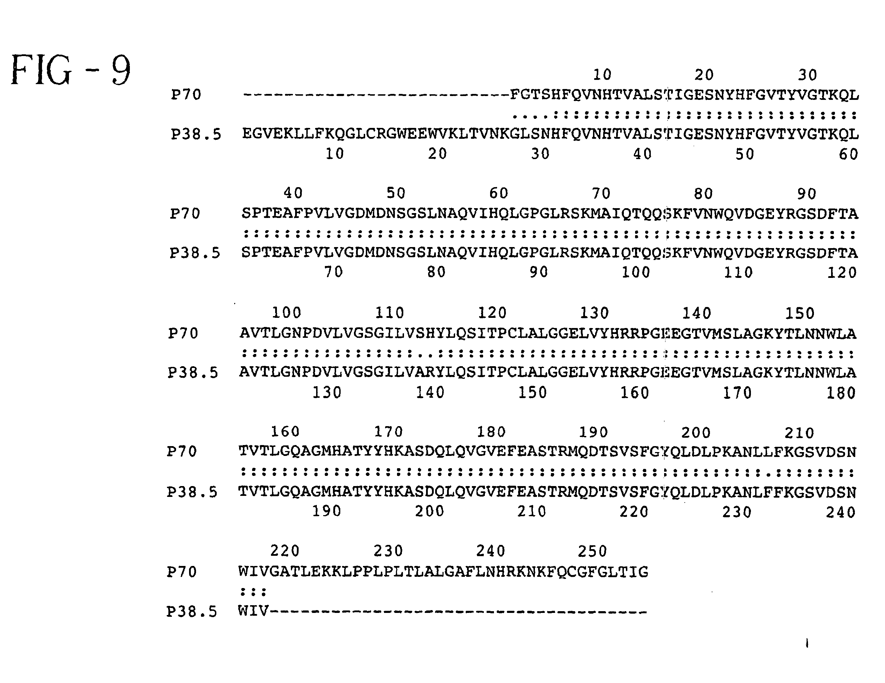

Figure 9 is an amino acid alignment of p38.5 and p70 cDNA fragments.

### DETAILED DESCRIPTION OF THE PREFERRED EMBODIMENT

In one embodiment, the present invention is an isolated and purified membrane protein having a molecular weight of about 38.5 kilodaltons (kD) (hereinafter designated "p38.5"), which is characteristically expressed by tumor cells that are susceptible to cell-mediated lysis by naive (non-activated) human natural killer (NK) cells and comprises the amino acid sequence defined by SEQ ID NO:3.

The protein designated p38.5 binds preferentially to NK cells in the presence of other types of lymphocytes. Lymphocytes include, in general B cells, T cells, and other large granular lymphocytes. As a result, the p38.5 protein can be used in methods of selective detection, identification, and separation of NK cells in biological samples, such as blood samples.

The p38.5 protein may be isolated from tumor cells that are susceptible to cell-mediated lysis by naive NK cells. In general, tumors or neoplasms include new growths of tissue in which the multiplication of cells is uncontrolled and progressive. Some such growths are benign, but others are termed "malignant," leading to death of the organism. Malignant neoplasms or "cancers" are distinguished from benign growths in that, in addition to exhibiting aggressive cellular proliferation, they invade surrounding tissues and metastasize. Moreover, malignant neoplasms are characterized in that they show a greater loss of differentiation (greater "dedifferentiation"), and of their organization relative to one another and their surrounding tissues. This property is also called "anaplasia."

Tumor cells susceptible to cell-mediated lysis by non-activated NK cells typically include hematologic tumors and the like. For example, leukemias. Leukemias include tumors derived from blood cell-forming tissues, including acute and chronic forms, and myeloid, lymphatic, and monocytic varieties. Other types of tumors, such as carcinomas, adenocarcinomas, and sarcomas are ordinarily resistant to NK cells. Carcinomas include those malignant neoplasms derived from epithelial cells which tend to infiltrate (invade) the surrounding tissues and give rise to metastases. Adenocarcinomas are carcinomas derived from glandular tissue or in which the tumor cells form recognizable glandular structures. Sarcomas broadly include tumors whose cells are embedded in a fibrillar or homogeneous substance like embryonic connective tissue.

The invention is particularly illustrated herein in reference to treatment of certain types of experimentally defined cancers. In these illustrative treatments, standard state-of-the-art *in vitro* models have been used. For example, K562 erythroleukemia cells, MOLT-4 cells, and Jurkat cells are all examples of well defined NK-susceptible tumors. These methods can be used to identify agents that can be expected to be efficacious in *in vivo* treatment regimens. However, it will be understood that treatment is not limited to these tumor types, but extends to any NK-susceptible tumor derived from any organ system.

**TABLE 1**

| **p38.5 Nucleotide Sequence** |
|---|
| |
| |
| |
| |
| |
| |
| |
| |
| |
| |
| |
| |
| |
| |
| |
| |

Nucleotide 33 of the p38.5 nucleotide sequence is either C or T, designated Y. Due to the degeneracy of the genetic code, no change results in the encoded protein. Nucleotide 412 of the p38.5 nucleotide sequence is either G or T, designated K. This difference results in two possible amino acid residues at position 138, either Ala or Ser. Nucleotide 416 of the p38.5 nucleotide sequence is either G or A, designated R. This difference results in two possible amino acid residues at position 139, either Arg or His. Alternative amino acid residues are indicated in parentheses below the respective codons.

Described herein is a vector such as a recombinant expression vector, containing a nucleic acid encoding the p38.5 protein. To illustrate, applicant has constructed a plasmid (designated "nahNKCRp38.5") comprising a 730 base pair fragment (SEQ ID NO:1) of the human p38.5 cDNA, as shown in Table 1. This fragment encodes an open reading frame of 243 amino acids (SEQ ID NO:2), as shown in Table 2. A microorganism deposit of nahNKCRp38.5 in compliance with the Budapest Treaty was made with the American Type Culture Collection (ATCC) located in Rockville, MD, on May 1, 1997, and assigned ATCC No. 98422.

**TABLE 2**

| **nahNKCRp38.5 Open Reading Frame-Amino Acid Sequence** |
|---|
| |
| |

Also described is a unique fragment of the p38.5 protein. Such fragments include antigenic or immunogenic regions. For example, applicant has employed an 11 mer peptide (SEQ ID NO:3) encoded by nucleotides 311 to 344 of the 730 base pair fragment, as shown Table 3. The 11mer is particularly suitable for the production of antibodies to p38.5. This 11 mer is also indicated in bold text in the sequence given in Table 2.

**TABLE 3**

| **Amino Acid Sequence of an internal peptide of K562 membrane protein, p38.5** |
|---|
| |

Described is also an isolated and purified functional membrane protein of about 70 kD found on naive human NK cells. This protein has been designated "p70". The 70 kD receptor binds to the cell surface ligand p38.5. The expression of p70 appears to mediate the destruction of tumor cells expressing p38.5.

Also described is a vector, e.g., a plasmid, comprising nucleic acid encoding some or all of the p70 protein. For example, applicant has demonstrated this embodiment by construcing a plasmid designated "nahNKRp70". A microorganism deposit of nahNKRp70 in compliance with the Budapest Treaty was made with the ATCC on May 1, 1997 and assigned ATCC No. 98424. Specifically, this plasmid contains a sequenced cDNA insert of 1.315 Kb determined from the 3' end of the gene, as shown in Table 4. This is SEQ ID NO:4.

**TABLE 4**

| **p70 Nucleotide Sequence** |
|---|
| |
| |
| |
| |
| |
| |
| |
| |
| |
| |
| |
| |
| |
| |
| |
| |
| |
| |

In the p70 sequence (SEQ ID NO:4), nucleotide 334 can be either T or G, designated K. As a result, amino acid residue 112 of the p70 protein may be Ser or Ala. Also in the p70 sequence, nucleotide 338 can be either A or G, designated R. As a result, amino acid residue 113 may be His or Arg. The alternative residues are given in parentheses. The stop codon is indicated by ***.

The amino acid sequence (SEQ ID NO:5) encoded by the sequenced region of the p70 cDNA is given in Table 5.

**TABLE 5**

| **nahNKRp70 Amino Acid Translation 5' End** |
|---|
| |

Described is also a bacteriophage (designated "nahNKL") containing cDNA encoding the entire naive human NK cell library constructed by subtractive hybridization with mRNA from human T-lymphocytes. The non-activated human NK cell library is contained in a bacteriophage vector designated nahNKL. A deposit of the library was made with the ATCC in compliance with the Budapest Treaty on May 1, 1997 and was assigned ATCC No. 98423. Specifically, the Lambda ZAP Express® vector (Stratagene, La Jolla, CA) was used to construct a library of cDNA inserts from human non-activated Natural Killer lymphocytes (NK cells). This library was prepared by subtractive hybridization with human T lymphocyte mRNA. The library is particularly useful for isolating and characterizing genes and gene products, in addition to p70, that are unique to NK cells as compared to T cells.

### Definition of Natural Killer Lymphocytes (NK Cells)

For general reference, a definition of natural killer cells is here provided. NK cells are non-T, non-B lymphocytes that are defined by the following properties: They have spontaneous lytic activity against cells infected with intra-cellular parasites (e.g. viruses) and certain types of tumor cells (usually of hematologic origin). They express various combinations of CD8, CD16 and CD56 on their surface and lack the surface expression of CD3 components, TCR heterodimers and immuynoglobulins (e.g., IgM, IgD.). They express cell surface molecules that regulate cytolytic activity by interaction with MHC class-1 molecules. They can be stimulated by incubation with cytokines, e.g. IL-2, to express lytic activity against a broader spectrum of target cells.

### Protein Methods

The p38.5 protein and the p70 protein can be isolated from mammals and insects. Mammals include, for example, humans and other primates, as well as pet animals such as dogs and cats, laboratory animals such as rats and mice, and farm animals such as horses, sheep, and cows.

The proteins and fragments described herein may be prepared by methods known in the art. Such methods include isolating the protein directly from cells, isolating or synthesizing DNA encoding the protein and using the DNA to produce recombinant protein, and synthesizing the protein chemically from individual amino acids.

Proteins can be isolated from a solubilized fraction by standard methods. Some suitable methods include precipitation and liquid chromatographic protocols such as ion exchange, hydrophobic interaction, and gel filtration. See, for example, Methods Enzymol (Guide to Protein Chemistry, Deutscher, ed., Section VII) pp. 182:309 (1990) and Scopes, Protein Purification, Springer-Verlag, New York (1987), which are herein incorporated by reference.

Alternatively, purified material is obtained by separating the protein on preparative SDS-PAGE gels, slicing out the band of interest and electroeluting the protein from the polyacrylamide matrix by methods known in the art. The detergent SDS is removed from the protein by known methods, such as by dialysis or the use of a suitable column, such as the Extracti-Gel column from Pierce:

The proteins may be chemically synthesized by methods known in the art. Suitable methods for synthesizing proteins are described by Stuart and Young, Solid Phase Peptide Synthesis, 2d ed., Pierce Chemical Company (1984).

The proteins may also be prepared by providing DNA that encodes the protein; amplifying or cloning the DNA in a suitable host; expressing the DNA in a suitable host; and harvesting the protein.

The proteins of the invention are isolated, which means that they are each essentially free of other proteins. Essentially free from other proteins means that the protein in question is at least 90%, preferably at least 95% and more preferably at least 98% free of other proteins.

Preferably, the proteins are each essentially pure, which means that the proteins are free not only of other proteins, but also of other materials used in the processes of isolation, identification, or purification of the proteins. Thus, the proteins are free of materials such as, for example, detergents, affinity binding agents and separation films. Detergents include sodium dodecyl sulfate and sarcosine. Affinity binding agents include agarose, avidin-agarose, streptavidin-agarose, biotin, and biotinylated proteins. Separation films include nitrocellulose paper and nitrocellulose/cellulose acetate paper. The protein is at least 90% free, preferably at least 95% free, and more preferably at least 98% free of such materials.

Mixtures of proteins can be separated by, for example, SDS-PAGE in accordance with the method of Laemmli, Nature 227:680-685 (1970). The molecular weights are determined by resolving single bands on SDS-PAGE and comparing their positions to those of known standards. The method is understood by those in the art to be accurate within a range of 3-5%. Molecular weights may vary slightly between determinations.

Determinations of whether two amino acid sequences are substantially homologous are, for the purpose of the present specification, based on FASTA searches in accordance with Pearson et al., Proc Natl Acad Sci USA 85:2444-2448 (1988). A substantially homologous sequence has at least 60% identity, preferably at least 85% identity and, more preferably, at least 95% identity in amino acid sequence.

In the present specification, the sequence of a first protein, such as a receptor or a ligand, or of a nucleic acid molecule that encodes the protein, is considered homologous to a second protein or nucleic acid molecule if the amino acid or nucleotide sequence of the first protein or nucleic acid molecule is at least about 30% homologous, preferably at least about 50% homologous, and more preferably at least about 65% homologous to the respective sequences of the second protein or nucleic acid molecule. In the case of proteins having high homology, the amino acid or nucleotide sequence of the first protein or nucleic acid molecule is at least about 75% homologous, preferably at least about 85% homologous, and more preferably at least about 95% homologous to the amino acid or nucleotide sequence of the second protein or nucleic acid molecule.

The proteins further include functional analogs. A protein is considered a functional analog of another protein for a specific function, as described herein, if the analog is immunologically cross-reactive with, and has the same function as, the other protein. The analog may, for example, be a fragment of the protein, or a substitution, addition, or deletion mutant of the protein.

As is also known, it is possible to substitute amino acids in a sequence with equivalent amino acids. Groups of amino acids known normally to be equivalent are:
(a) Ala (A), Ser (S), Thr (T), Pro (P), Gly (G);
(b) Asn (N), Asp (D), Glu (E), Gln (Q);
(c) His (H), Arg (R), Lys (K);
(d) Met (M), Leu (L), Ile (I), Val (V); and
(e) Phe (F), Tyr (Y), Trp (W).

Substitutions, additions, and/or deletions in the antigenic sequences may be made as long as the proteins continue to satisfy the immunological criteria described herein. An amino acid sequence that is substantially the same ass another sequence, but that differs from the other sequence by means of one or more substitutions, additions and/or deletions is considered to be an equivalent sequence. Preferably, less than 25%, more preferably less than 10%, of the number of amino acid residues in a sequence are substituted for, added to, or deleted from the fragments in the proteins.

A protein may be considered to be homologous to the protein of the invention if nucleic acids encoding the two proteins hybridize with one another. A higher degree of homology is shown if the hybridization occurs under stringent hybridization conditions. Control of hybridization conditions, and the relationships between hybridization conditions and degree of homology are understood by those skilled in the art. See, e.g., Sambrook J, Fritsch EF, and Maniatis T, Molecular Cloning. A Laboratory Manual, 2d ed., Cold Spring Harbor Laboratory, Cold Spring Harbor (1989). Thus, a homologous protein may be a protein that is encoded by a nucleic acid that hybridizes with a nucleic acid encoding a protein of the invention under stringent hybridization conditions.

It may be desirable that such structurally homologous proteins will also exhibit functional homology, insofar as the homologous protein has substantially the same function as a protein of the invention. For example, structurally homologous proteins may be considered to be functionally homologous if they exhibit similar binding of a ligand, or similar immunogenicity, etc. In the present case, given the demonstrated affinity of the p38.5 and p70 proteins for one another, functionally homologous proteins may exhibit similar binding affinities.

However, it is known that two proteins or two nucleic acids may be considered to be substantially homologous in structure, and yet differ substantially in function. For example, single nucleotide polymorphisms (SNPs) among alleles may be expressed as proteins having substantial differences in antibody-binding or ligand-binding activity. Other structural differences, such as substitutions, deletions, splicing variants, and the like, may affect the function of otherwise structurally identical or homologous proteins. Allelic variations in the p38.5 protein are within the scope of the invention.

The protein of the invention may be an entire p38.5 protein as they may be isolated from natural sources, or an antigenic, preferably immunogenic, fragment of the whole protein. Antigenic and/or immunogenic fragments of antigenic and/or immunogenic proteins may be identified by methods known in the art. By antigenic, it is meant that the protein induces specific antibodies in a mammal or specifically binds to such antibodies. By immunogenic is meant that the protein induces or participates in generation of an immune response.

Fragments containing antigenic sequences may be selected on the basis of generally accepted criteria of potential antigenicity and/or exposure. Such criteria include the hydrophilicity and relative antigenic index, as determined by surface exposure analysis of proteins. The determination of appropriate criteria is known to those skilled in the art, and has been described, for example, by Hopp et al., Proc Natl Acad Sci USA 78:3824-3828 (1981); Kyte et al., J Mol Biol 157:105-132 (1982); Emini, J Virol 55:836-839 (1985); Jameson et al., CA BIOS 4:181-186 (1988); and Karplus et al., Naturwissenschaften 72:212-213 (1985). Amino acid domains predicted by these criteria to be surface exposed are selected preferentially over domains predicted to be more hydrophobic or hidden.

Methods for isolating and identifying antigenic fragments from known antigenic proteins are described by Salfeld et al., J Virol 63:798-808 (1989) and by Isola et al., J Virol 63:2325-2334 (1989).

The protein of the invention may be expressed in the form of a fusion protein with an appropriate fusion partner. The fusion partner preferably facilitates purification and identification. Increased yields may be achieved when the fusion partner is expressed naturally in the host cell. Some useful fusion partners include beta-galactosidase (Gray et al., Proc Natl Acad Sci USA 79:6598 (1982)); trpE (Itakura et al., Science 198:1056 (1977)); protein A (Uhlen et al., Gene 23:369 (1983)); glutathione S-transferase (Johnson, Nature 338:585 (1989)); Van Etten et al., Cell 58:669 (1989)); and maltose binding protein (Guan et al., Gene 67:21-30 (1987); Maina et al., Gene 74:36-373 (1988); Riggs, P., in Ausebel, F.M. et al (eds) Current Protocols in Molecular Biology, Greene Associates/Wiley Interscience, New York (1990)).

Such fusion proteins may be purified by affinity chromatography using reagents that bind to the fusion partner. The reagent may be a specific ligand of the fusion partner or an antibody, preferably a monoclonal antibody. For example, fusion proteins containing beta-galactosidase may be purified by affinity chromatography using an anti-beta-galactosidase antibody column (Ullman, Gene 29:27-31 (1984)). Similarly, fusion proteins containing maltose binding protein may be purified by affinity chromatography using a column containing cross-linked amylose; see Guan, European Patent Application 286,239.

The protein may occur at the amino-terminal or the carboxy-terminal side of the cleavage site. Optionally, the DNA that encodes the fusion protein is engineered so that the fusion protein contains a cleavable site between the protein and the fusion partner. Both chemical and enzymatic cleavable sites are known in the art. Suitable examples of sites that are cleavable enzymatically include sites that are specifically recognized and cleaved by collagenase (Keil et al., FEBS Letters 56:292-296 (1975)); enterokinase (Hopp et al., Bio/Technology 6:1204-1210 (1988)); factor Xa (Nagai et al., Methods Enzymol 153:461-481 (1987)); and thrombin (Eaton et al., Biochemistry 25:505 (1986)). Collagenase cleaves between proline and X in the sequence Pro-X-Gly-Pro wherein X is a neutral amino acid. Enterokinase cleaves after lysine in the sequence Asp-Asp-Asp-Asp-Lys. Factor Xa cleaves after arginine in the sequence Ile-Glu-Gly-Arg. Thrombin cleaves between arginine and glycine in the sequence Arg-Gly-Ser-Pro.

Specific chemical cleavage agents are also known. For example, cyanogen bromide cleaves at methionine residues in proteins.

The recombinant protein is purified by methods known in the art. Such methods include affinity chromatography using specific antibodies. Alternatively, the recombinant protein may be purified using a combination of ion-exchange, size-exclusion, and hydrophobic interaction chromatography using methods known in the art. These and other suitable methods are described, for example, by Marston, "The purification of eukaryotic proteins expressed in *E. coli,"* in DNA Cloning, DM Glover, ed., Volume III, IRL Press Ltd., England (1987); Marston FAO and Hartley DL, "Solubilization of protein aggregates," pp. 266-267 in Guide to Protein Purification, Deutscher MP, ed., Academic Press, San Diego (1990).

### Nucleic Acids Methods

"Nucleic acid," as used herein, means any deoxyribonucleic acid (DNA) or ribonucleic acid (RNA) that encodes in its nucleotide sequence a protein as described herein or a unique fragment thereof. The fragment can be an oligonucleotide (i.e., about 8 to about 50 nucleotides in length) or a polynucleotide (about 50 to about 2,000 or more nucleotides in length). For example, nucleic acids include messenger RNA (mRNA), complementary DNA (cDNA), genomic DNA, synthetic DNA or RNA, and the like. The nucleic acid can be single stranded, or partially or completely double stranded (duplex). Duplex nucleic acids can be homoduplex or heteroduplex.

Specifically described are nucleic acids that have a nucleotide sequence including the sequence defined by SEQ ID NO:1, or a homolog thereof, or unique fragments thereof. In the present specification, the sequence of a nucleic acid molecule that encodes the protein is considered homologous to a second nucleic acid molecule if the nucleotide sequence of the first nucleic acid molecule is at least about 30% homologous, preferably at least about 50% homologous, and more preferably at least about 65% homologous to the sequence of the second nucleic acid molecule. In the case of nucleic acids having high homology, the nucleotide sequence of the first nucleic acid molecule is at least about 75% homologous, preferably at least about 85% homologous, and more preferably at least about 95% homologous to the nucleotide sequence of the second nucleic acid molecule. For example, a test for homology of two nucleic acid sequences is whether they hybridize under normal hybridization conditions, preferably under stringent hybridization conditions.

As noted, described are also fragments of nucleic acids encoding the p38.5 protein or the p70 protein Such fragments include primers and probes which are useful as tools in numerous molecular engineering techniques. The fragment can be used as a primer ("amplimer") to selectively amplify nucleic acid, such as genomic DNA, total RNA, etc. The fragment can also be an oligonucleotide complementary to a target nucleic acid molecule, i.e:, the fragment can be a probe. In either case, the oligonucleotide can be RNA or DNA.

The length of the oligonucleotide probe is not critical, as long as it is capable of hybridizing to the target molecule. The oligonucleotide should contain at least 6 nucleotides, preferably at least 10 nucleotides, and more preferably at least 15 nucleotides. It is preferred that the probe hybridize to a sequence that is unique to the sequence encoding the p38.5 protein or the p70 protein. Such probes are said to hybridize specifically with their target sequences. There is no upper limit to the length of the oligonucleotide probes. Longer probes are more difficult to prepare and require longer hybridization times. Therefore, the probe should not be longer than necessary. Normally, the oligonucleotide probe will not contain more than 50 nucleotides, preferably not more than 40 nucleotides, and more preferably not more than 30 nucleotides.

Oligonucleotide probes and primers may be provided as homogeneous preparations of identical oligonucleotides, or as heterogeneous preparations of non-identical and/or non-overlapping sets of oligonucleotides. For example, in amplification processes, pairs of primers may be used that are complementary to the portions of the two strands of double stranded DNA.

In any case, methods for making and using nucleic acid probes are well documented in the art. For example, see Keller GH and Manak MM, DNA Probes, 2d ed., Macmillan Publishers Ltd., England (1991) and Hames BD and Higgins SJ, eds., Gene Probes I and Gene Probes II, IRL Press, Oxford (1995).

The DNA may be synthesized chemically from the four nucleotides in whole or in part by methods known in the art. Such methods include those described by Caruthers MH, Science 230:281-285 (1985). DNA can also be synthesized by preparing overlapping double-stranded oligonucleotides, filling in the gaps, and ligating the ends together. See, generally, Sambrook et al. (1989) and Glover DM and Hames BD, eds., DNA Cloning, 2d ed., Vols. 1-4, IRL Press, Oxford (1995).

The DNA may be cloned in a suitable host cell and expressed. The DNA and protein may be recovered from the host cell. See, generally, Sambrook et al. (1989), for methods relating to the manufacture and manipulation of nucleic acids.

Methods well known in the are can be used for isolating DNA once the protein has been isolated and purified. Many of these methods are described in Sambrook et al. (1989). In this way the full nucleic acid sequence of the gene for the proteins can be obtained. The immunological screening method is a preferred method.

For example, chromosomal DNA is isolated and cleaved into fragments of suitable size by standard methods. Suitable DNA cleavage methods include, for example, sonication and the use of restriction endonucleases. A suitable average fragment size is approximately 500 bp.

Linkers are added to the fragments and the resulting fragments are ligated into a suitable vector. The linker corresponds to a restriction site in the vector. Suitable linkers include, for example, *Eco*RI*, Pst*I and *Bam*HI*.* A suitable vector is lambda-gt11. Ligated DNA may be packaged by commercial kits, such as a kit manufactured by Promega.

Proteins from the resulting library are expressed in a suitable host, typically *E. coli.* The plaques that are obtained are screened immunologically by methods known in the art. Sambrook et al. (1989). Screening may be facilitated by the use of a commercial screening kit, such as the picoBlue Immunological Screening Kit of Stratagene (La Jolla, CA) in accordance with the manufacturer's instructions.

Plaques that bind the protein-specific antibody are selected from non-reacting plaques and purified. Sambrook et al. (1989). The DNA from purified phage is isolated by methods known in the art. Suitable methods include, for example, polyethylene glycol precipitation, phage lysis, and anion exchange chromatography, which can be facilitated by the use of a kit manufactured by Qiagen (Studio City, CA).

The fragments described above, or sub-fragments of them, can be used as probes for obtaining additional fragments of the protein gene.

By suitable extensions of the fragments, the entire gene is sequenced. The limits of the coding sequence are determined by methods known in the art, such as by insertional mutagenesis.

DNA encoding the proteins can be isolated by an immunoscreening method. Such methods are described by Sambrook et al. (1989).

DNA encoding the proteins is isolated by using the partial amino acid sequence of the coding region SEQ ID NO:2 and SEQ ID NO:5 provided above or a fragment thereof to prepare one or more oligonucleotide probes. The probe is labeled and used to screen a genomic or cDNA library in a suitable vector, such as in phage lambda. The cDNA library may by prepared from mRNA by known methods, such as those described by Gubler and Hoffman, Gene 25:263-270 (1983).

The DNA isolated is sequenced, and the sequence used to prepare additional oligonucleotide probes. This procedure may be repeated to obtain overlapping fragments until a complete open reading frame is produced.

Given the nucleic acid sequence disclosed herein, the artisan can further design nucleic acid structures having particular functions in various types of applications. For example, the artisan can construct oligonucleotides or polynucleotides for use as primers in nucleic acid amplification procedures, such as the polymerase chain reaction (PCR), ligase chain reaction (LCR), Repair Chain Reaction (RCR), PCR oligonucleotide ligation assay (PCR-OLA), and the like. Oligonucleotides useful as probes in hybridization studies, such as *in situ* hybridization, can be constructed. Numerous methods for detectably labeling such probes with radioisotopes, fluorescent tags, enzymes, binding moieties (e.g., biotin), and the like are known, so that the probes can be adapted for easy detectability.

Oligonucleotides can also be designed and manufactured for other purposes. For example, the invention enables the artisan to design antisense oligonucleotides, and triplex-forming oligonucleotides, and the like, for use in the study of structure/function relationships. Homologous recombination can be implemented by adaptation of the nucleic acid of the invention for use as targeting means.

As new and specific nucleotide sequences are disclosed herein, the artisan will recognize that the nucleic acids can be produced by any synthetic or recombinant process such as is well known in the art. Nucleic acids according to the invention can further be modified to alter biophysical or biological properties by means of techniques known in the art. For example, the nucleic acid can be modified to increase its stability against nucleases (e.g., "end-capping"), or to modify its lipophilicity, solubility, or binding affinity to complementary sequences. Methods for modifying nucleic acids to achieve specific purposes are disclosed in the art, for example, in Sambrook et al. (1989). Moreover, the nucleic acid can include one or more portions of nucleotide sequence that are non-coding for the protein of interest.

The skilled artisan appreciates that, if an amino acid sequence (primary structure) is known, a family of nucleic acids can then be constructed, each having a sequence that differs from the others by at least one nucleotide, but where each different nucleic acid still encodes the same protein. For example, if a protein has been sequenced but its corresponding gene has not been identified, the gene can be acquired through amplification of genomic DNA using a set of degenerate primers that specify all possible sequences encoding the protein.

The entire gene or additional fragments of the gene are preferably isolated by using the known DNA sequence or a fragment thereof as a probe. To do so, restriction fragments from a genomic or cDNA library are identified by Southern hybridization using labeled oligonucleotide probes derived from SEQ ID NO:1.

Methods for determining whether a nucleic acid molecule probe recognizes a specific nucleic acid molecule in a sample are known in the art. Generally, a labeled probe that is complementary to a nucleic acid sequence suspected of being in a sample is prepared. Preferably, the target nucleic acid molecule is immobilized. The presence of probe hybridized to the target nucleic acid molecule indicates the presence of the nucleic acid molecule in the sample. Examples of suitable assay methods are described by Dallas et al., "The characterization of an Escherichia coli plasmid determinant that encodes for the production of a heat-labile enterotoxin," in K. N. Timmis and A. Puehler, eds., Plasmids of Medical, Environmental, and Commercial Importance, Elsevier/North-Holland Publishing Co., Amsterdam, pages 113-122 (1975); Grunstein and Hogness, Proc Natl Acad Sci USA 72:3961-3965 (1975); Palva et al. in U.S. Patent No. 4,731,325; Mullis et al. in U.S. Patent No. 4,683,195; Schneider et al. in U.S. Patent No. 4,882,269; and Segev in PCT Application WO 90/01069.

The DNA obtained may be amplified by methods known in the art. One suitable method is the polymerase chain reaction (PCR) method described by Saiki et al., Science 239:487 (1988), Mullis et al in U.S. Patent No. 4,683,195 and by Sambrook et al. (1989). It is convenient to amplify the clones in the lambda-gt10 or lambda-gt11 vectors using lambda-gt10 or lambda-gt11-specific oligomers as the amplimers (available from Clontech, Palo Alto, CA).

Larger synthetic nucleic acid structures can also be manufactured having specific and recognizable utilities according to the invention. For example, vectors (e.g., recombinant expression vectors) are known which permit the incorporation of nucleic acids of interest for cloning and transformation of other cells. Thus, the invention further includes vectors (e.g., plasmids, phages, cosmids, etc.) which incorporate the nucleotide sequence of the invention, especially vectors which include the gene for expression of the protein encoded by the nucleic acid of the invention.

DNA encoding a protein of the invention may be replicated and used to express recombinant protein following insertion into a wide variety of host cells in a wide variety of cloning and expression vectors. The host may be prokaryotic or eukaryotic. The DNA may be obtained from natural sources and, optionally, modified. The genes may also be synthesized in whole or in part.

Cloning vectors may comprise segments of chromosomal, non-chromosomal and synthetic DNA sequences. Some suitable prokaryotic cloning vectors include plasmids from *E. coli,* such as colE1, pCR1, pBR322, pMB9, pUC, pKSM, and RP4. Prokaryotic vectors also include derivatives of phage DNA such as M13 fd, and other filamentous single-stranded DNA phages.

Vectors for expressing proteins in bacteria, especially *E. coli,* are also known. Such vectors include the pK233 (or any of the *tac* family of plasmids), T7, and lambda P_{L}. Examples of vectors that express fusion proteins are PATH vectors described by Dieckmann and Tzagoloff, J Biol Chem 260:1513-1520 (1985). These vectors contain DNA sequences that encode anthranilate synthetase (TrpE) followed by a polylinker at the carboxy terminus. Other expression vector systems are based on beta-galactosidase (pEX); maltose binding protein (pMAL); glutathione S-transferase (pGST). See, e.g, Smith DB, Gene 67:31-40 (1988) and Abath FG, Peptide Research 3(4):167-168 (1990).

Vectors useful for cloning and expression in yeast are available. A suitable example is the 2µ circle plasmid.

Suitable cloning/expression vectors for use in mammalian cells are also known. Such vectors include well-known derivatives of SV-40, adenovirus, cytomegalovirus (CMV) retrovirus-derived DNA sequences. Any such vectors, when coupled with vectors derived from a combination of plasmids and phage DNA, i.e., shuttle vectors, allow for the isolation and identification of protein coding sequences in prokaryotes.

Further eukaryotic expression vectors are known in the art (e.g., PJ Southern and P Berg, J Mol Appl Genet 1:327-341 (1982); Subramani et al. (1981); RJ Kaufmann and PA Sharp, "Amplification and expression of sequences cotransfected with a modular dihydrofolate reductase complementary DNA gene," J Mol Biol 159:601-621 (1982); RJ Kaufmann and PA Sharp, Mol Cell Biol 159:601-664 (1982); SI Scahill et al., "Expression and characterization of the product of a human immune interferon DNA gene in Chinese hamster ovary cells," Proc Natl Acad Sci USA 80:4654-4659 (1983); G Urlaub and LA Chasin, Proc Natl Acad Sci USA 77:4216-4220 (1980).

The expression vectors useful in the present invention contain at least one expression control sequence that is operatively linked to the DNA sequence or fragment to be expressed. The control sequence is inserted in the vector in order to control and to regulate the expression of the cloned DNA sequence. Examples of useful expression control sequences are the *lac* system, the *trp* system, the *tac* system, the *trc* system, major operator and promoter regions of phage lambda, the control region of fd coat protein, the glycolytic promoters of yeast, e.g., the promoter for 3-phosphoglycerate kinase, the promoters of yeast acid phosphatase, e.g., Pho5, the promoters of the yeast alpha-mating factors, and promoters derived from polyoma, adenovirus, retrovirus, and simian virus, e.g., the early and late promoters or SV40, and other sequences known to control the expression of genes of prokaryotic or eukaryotic cells and their viruses or combinations thereof.

Useful expression hosts include well-known prokaryotic and eukaryotic cells. Some suitable prokaryotic hosts include, for example, *E. coli,* such as *E. coli* SG-936, *E. coli* HB 101, *E. coli* W3110, *E. coli* X1776, *E. coli* X2282, *E. coli* DHI, and *E. coli* MRCl, *Pseudomonas* sp., *Bacillus* sp., such as *B. subtilis,* and *Streptomyces* sp. Suitable eukaryotic cells include yeasts and other fungi, insect, animal cells, such as COS cells and CHO cells, human cells and plant cells in tissue culture.

The restriction fragments are cloned into a suitable vector, such as a plasmid or bacteriophage, and sequenced in accordance with methods known in the art. A suitable sequencing method is the dideoxy chain terminating method described by Sanger et al., Proc Natl Acad Sci USA 74:5463-5467 (1977). Suitable vectors and polymerases for sequencing are known. A suitable vector is the Bluescript vector available from Stratagene. A suitable polymerase is Sequenase (United States Biochemical Corp., Cleveland, OH).

A variety of methods are available to transfer genes into cells. For example, a method for transferring genes between cells is described in Phelan et al., Nature Biotechnology 16: 440-443 (1998).

DNA expressing functional homologs of the protein can be prepared from wild-type DNA by site-directed mutagenesis. See, for example, Zoller MJ, and Smith M, Nucleic Acids Res 10:6487-6500 (1982); Zoller MJ, Methods Enzymol 100:468-500 (1983); Zoller MJ, DNA 3(6):479-488 (1984); and McPherson MJ, ed., Directed Mutagenesis: A Practical Approach, IRL Press, Oxford (1991).

The present invention also includes isolated nucleic acid molecules that encode any of the proteins of the invention described above. The nucleic acid molecule may be DNA or RNA.

The nucleic acid molecules may be used as probes for detecting DNA encoding the protein, as explained below, or to produce a protein of the invention, as explained above. The nucleic acid molecule may be prepared by methods known in the art. Suitable methods include isolating the DNA from cells or chemically synthesizing the DNA in accordance with known procedures as described above.

### Antibody Methods

The present invention further provides antibodies raised against a protein of the invention. An "antibody" in accordance with the present specification is defined broadly as a protein that binds specifically to an epitope. The antibody may be polyclonal or monoclonal. Antibodies further include recombinant polyclonal or monoclonal Fab fragments prepared in accordance with the method of Huse et al., Science 246:1275-1281 (1989).

Polyclonal antibodies are isolated from mammals that have been inoculated with the protein or a functional analog in accordance with methods known in the art. Briefly, polyclonal antibodies may be produced by injecting a host mammal, such as a rabbit, mouse, rat, or goat, with the protein or a fragment thereof capable of producing antibodies that distinguish between mutant and wild-type protein. The peptide or peptide fragment injected may contain the wild-type sequence or the mutant sequence. Sera from the mammal are extracted and screened to obtain polyclonal antibodies that are specific to the peptide or peptide fragment.

The antibodies are preferably monoclonal. Monoclonal antibodies may be produced by methods known in the art. These methods include the immunological method described by Kohler and Milstein, Nature 256:495-497 (1975) and by Campbell, in Burdon et al., eds, Laboratory Techniques in Biochemistry and Molecular Biology, Vol. 13, Elsevier Science Publishers, Amsterdam (1985); as well as the recombinant DNA method described by Huse et al., Science 246:1275-1281 (1989).

To produce monoclonal antibodies, a host mammal is inoculated with a peptide or peptide fragment as described above, and then boosted. Spleens are collected from inoculated mammals a few days after the final boost. Cell suspensions from the spleens are fused with a tumor cell in accordance with the general method described by Kohler and Milstein (1975). See also Campbell (1985). To be useful, a peptide fragment must contain sufficient amino acid residues to define the epitope of the molecule being detected.

If the fragment is too short to be immunogenic, it may be conjugated to a carrier molecule. Some suitable carrier molecules include keyhole limpet hemocyanin and bovine serum albumin. Conjugation may be carried out by methods known in the art. One such method is to combine a cysteine residue of the fragment with a cysteine residue on the carrier molecule.

Methods for preparing polyclonal and monoclonal antibodies that exhibit specificity toward single amino acid differences between oncogenes are described by McCormick et ai., U.S. Patent No. 4,798,787.

Antibodies can be engineered using genetic techniques to produce chimeric antibodies including protein components from two or more species. For use in *in vivo* applications with human subject, the antibody can be humanized, containing an antigen binding region from, e.g., a rodent, with the bulk of the antibody replaced with sequences derived from human immunoglobulin. Methods are also known for inducing expression of engineered antibodies in various cell types, such as mammalian and microbial cell types. Numerous techniques for preparing engineered antibodies are described, e.g., in Owens RJ and Young RJ, "The genetic engineering of monoclonal antibodies," J Immunol Meth 168:149-165 (1994).

Assays for directly detecting the presence of proteins on or in cells with antibodies follow known formats, such as, fluorescent activated flow cytometry, fluorescent microscopy, and immuno-electron microscopy. Moreover, in general assays for detecting the presence of proteins with antibodies have been previously described and follow known formats, such as standard blot and ELISA formats. These formats are normally based on incubating an antibody with a sample suspected of containing the protein and detecting the presence of a complex between the antibody and the protein. The antibody is labeled either before, during, or after the incubation step. The protein is preferably immobilized prior to detection. Immobilization may be accomplished by directly binding the protein to a solid surface, such as a microtiter well, or by binding the protein to immobilized antibodies.

In a preferred embodiment, a protein is immobilized on a solid support through an immobilized first antibody specific for the protein. The immobilized first antibody is incubated with a sample suspected of containing the protein. If present, the protein binds to the first antibody.

A second antibody, also specific for the protein, binds to the immobilized protein. The second antibody may be labeled by methods known in the art. Non-immobilized materials are washed away, and the presence of immobilized label indicates the presence of the protein. This and other immunoassays are described by David et al., U.S. Patent No. 4,376,110.

Immunoassays may involve one step or two steps. In a one-step assay, the target molecule, if it is present, is immobilized and incubated with a labeled antibody. The labeled antibody binds to the immobilized target molecule. After washing to remove unbound molecules, the sample is assayed for the presence of the label.

In a two-step assay, immobilized target molecule is incubated with an unlabeled first antibody. The target molecule-antibody complex, if present, is then bound to a second, labeled antibody that is specific for the unlabeled antibody. The sample is washed and assayed for the presence of the label, as described above.

The immunometric assays described above include simultaneous sandwich, forward sandwich, and reverse sandwich immunoassays. These terms are well known to those skilled in the art.

In a forward sandwich immunoassay, a sample is first incubated with a solid phase immunoabsorbent containing antibody against the protein. Incubation is continued for a period of time sufficient to allow the protein in the sample to bind to the immobilized antibody in the solid phase. After the first incubation, the solid phase immunoabsorbent is separated from the incubation mixture and washed to remove excess protein and other interfering substances which also may be present in the sample. Solid phase immunoabsorbent-containing protein bound to the immobilized antibodies is subsequently incubated for a second time with soluble labeled antibody cross-reactive with a different domain on the protein. After the second incubation, another wash is performed to remove the unbound labeled antibody from the solid immunoabsorbent and to remove non-specifically bound labeled antibody. Labeled antibody bound to the solid phase immunoabsorbent is then detected and the amount of labeled antibody detected serves as a direct measure of the amount of antigen present in the original sample. Alternatively, labeled antibody that is not associated with the immunoabsorbent complex can also be detected, in which case the measure is in inverse proportion to the amount of antigen present in the sample. Forward sandwich assays are described, for example, in U.S. Patent Nos. 3,867,517, 4,012,294, and 4,376,110.

In a reverse sandwich assay, the sample is initially incubated with labeled antibody. The solid phase immunoabsorbent containing immobilized antibody cross-reactive with a different domain on the protein is added the labeled antibody, and a second incubation is carried out. The initial washing step required by a forward sandwich assay is not required, although a wash is performed after the second incubation. Reverse sandwich assays have been described, for example, in U.S. Patent Nos. 4,098,876 and 4,376,110.

In a simultaneous sandwich assay, the sample, the immunoabsorbent with immobilized antibody, and labeled soluble antibody specific to a different domain are incubated simultaneously in one incubation step. The simultaneous assay requires only a single incubation and does not require any washing steps. The use of a simultaneous assay is a very useful technique, providing ease of handling, homogeneity, reproducibility, linearity of the assays, and high precision. See U.S. Patent No. 4,376,110 to David et al.

In each of the above assays, the sample containing antigen, solid phase immunoabsorbent with immobilized antibody and labeled soluble antibody are incubated under conditions and for a period of time sufficient to allow antigen to bind to the immobilized antibodies and to the soluble antibodies. In general, it is desirable to provide incubation conditions sufficient to bind as much antigen as possible, since this maximizes the binding of labeled antibody to the solid phase, thereby increasing the signal. The specific concentrations of labeled and immobilized antibodies, the temperature and time of incubation, as well as other such assay conditions, can be varied, depending upon various factors including the concentration of antigen in the sample, the nature of the sample an the like. Those skilled in the art will be able to determine operative and optimal assay conditions for each determination by employing routine experimentation.

There are many solid phase immunoabsorbents which have been employed and which can be used in the present invention. Well known immunoabsorbents include beads formed from glass, polystyrene, polypropylene, dextran, nylon, and other material; and tubes formed from or coated with such materials, and the like. The immobilized antibodies may be covalently or physically bound to the solid phase immunoabsorbent, by techniques such as covalent bonding via an amide or ester linkage or by absorption.

The protein of the invention may be used to detect the presence of antibodies specific for the protein in a sample.

The proteins may be labeled and used as probes in standard immunoassays to detect antibodies against the proteins in samples, such as in the sera or other bodily fluids of patients being tested for the presence ofNK-susceptible tumors or NK cells. In general, a protein in accordance with the invention is incubated with the sample suspected of containing antibodies to the protein. The protein is labeled either before, during, or after incubation. The detection of labeled protein bound to an antibody in the sample indicates the presence of the antibody. The antibody is preferably immobilized.

Suitable assays are known in the art, such as the standard ELISA protocol described by RH Kenneth, "Enzyme-linked antibody assay with cells attached to polyvinyl chloride plates" in Kenneth et al., Monoclonal Antibodies, Plenum Press, New York, pp. 376 et seq. (1981).

Briefly, plates are coated with antigenic protein at a concentration sufficient to bind detectable amounts of the antibody. After incubating the plates with the protein, the plates are blocked with a suitable blocking agent, such as, for example, 10% normal goat serum. The sample, such as patient sera, is added and titered to determine the endpoint. Positive and negative controls are added simultaneously to quantitate the amount of relevant antibody present in the unknown samples. Following incubation, the samples are probed with goat anti-human Ig conjugated to a suitable label, such as an enzyme. The presence of anti-protein antibodies in the sample is indicated by the presence of the label.

For use in immunoassays, the probe may be the entire protein or may be a functional analog thereof. Functional analogs of these proteins include fragments and substitution; addition and deletion mutations that do not destroy the ability of the proteins to bind to their antibodies. As long as the proteins are able to detect antibodies specific for the protein, they are useful in the present invention.

### Labeled Probes

The nucleic acid or protein (including antibody) probes described herein can be detectably labeled in accordance with methods known in the art. In general, the probe can be modified by attachment of a detectable label moiety to the probe, or a detectable probe can be manufactured with a detectable label moiety incorporated therein. The detectable label moiety can be any detectable moiety, many of which are known in the art, including radioactive atoms, electron dense atoms, enzymes, chromogens and colored compounds, fluorogens and fluorescent compounds, members of specific binding pairs, and the like.

Methods for labeling oligonucleotide probes have been described, for example, by Leary et al., Proc Natl Acad Sci USA (1983) 80:4045; Renz and Kurz, Nucl Acids Res 12:3435 (1984); Richardson and Gumport, Nucl Acids Res 11:6167 (1983); Smith et al., Nucl Acids Res 13:2399 (1985); Meinkoth and Wahl, Anal Biochem 138:267 (1984). Other methods for labeling nucleic acids are described, for example, in U.S. Patent Nos. 4,711,955, 4,687,732, 5,241,060, 5,244,787, 5,328,824, 5,580,990, and 5,714,327.

Methods for labeling antibodies have been described, for example, by Hunter et al. (1962) and by David et al., Biochemistry 13:1014-1021 (1974). Additional methods for labeling antibodies have been described in U.S. Patent Nos. 3,940,475 and 3,645,090.

The label moiety may be radioactive. Some examples of useful radioactive labels include ³²P, ¹²⁵I*,* ¹³¹I, and ³H. Use of radioactive labels have been described in U.K. patent document No. 2,034,323, U.S. Patent Nos. 4,358,535. and 4,302,204.

Some examples of non-radioactive labels include enzymes, chromogens, atoms and molecules detectable by electron microscopy, and metal ions detectable by their magnetic properties.

Some useful enzymatic labels include enzymes that cause a detectable change in a substrate. Some useful enzymes (and their substrates) include, for example, horseradish peroxidase (pyrogallol and o-phenylenediamine), beta-galactosidase (fluorescein beta-D-galactopyranoside), and alkaline phosphatase (5-bromo-4-chloro-3-indolyl phosphate/nitro blue tetrazolium). The use of enzymatic labels has been described, for example, in U.K. 2,019,404, EP 63,879, and by Rotman, Proc Natl Acad Sci USA 47:1981-91 (1961).

Useful reporter moieties include, for example, fluorescent, phosphorescent, chemiluminescent, and bioluminescent molecules, as well as dyes. Some specific colored or fluorescent compounds useful in the present invention include, for example, fluoresceins, coumarins, rhodamines, Texas red, phycoerythrins, umbelliferones, LUMINOL®, and the like. Chromogens or fluorogens, i.e., molecules that can be modified (e.g., oxidized) to become colored or fluorescent or to change their color or emission spectra, are also capable of being incorporated into probes to act as reporter moieties under particular conditions.

The label moieties may be conjugated to the probe by methods that are well known in the art. The label moieties may be directly attached through a functional group on the probe. The probe either contains or can be caused to contain such a functional group. Some examples of suitable functional groups include, for example, amino, carboxyl, sulfhydryl, maleimide, isocyanate, and isothiocyanate.

Alternatively, label moieties such as enzymes and chromogens may be conjugated to antibodies or nucleotides by means of coupling agents, such as dialdehydes, carbodiimides, dimaleimides, and the like.

The label moiety may also be conjugated to the probe by means of a ligand attached to the probe by a method described above and a receptor for that ligand attached to the label moiety. Any of the known ligand-receptor binding pair combinations is suitable. Some suitable ligand-receptor pairs include, for example, biotin-avidin or -streptavidin, and antibody-antigen. The biotin-avidin combination may be preferred.

### Methods of Use

Diagnostic reagents and therapeutic agents can be derived from the above-described genes or gene products. One preferred diagnostic method includes the use of an antibody or fragment thereof that has specific binding affinity for one of the expressed gene products p38.5 or p70, or characteristic fragments thereof. One such antibody is an antibody raised against an internal 11 mer peptide found within the p38.5 (SED ID NO:3).

Therapeutic agents include, for example, cytotoxic agents bound to the p38.5 protein, the p70 protein, or fragments thereof. Other therapeutic agents include antibodies or fragments raised against the p38.5 protein, the p70 protein or fragments thereof. Antibodies may be bound to cytotoxic agents for targeted delivery of the cytotoxic agents to NK-susceptible tumors or NK cells. In fact, p38.5 alone can be used for immunosuppression since this protein significantly reduces NK cell activity as demonstrated herein.

The diagnostic reagents and methods and the therapeutic agents can be used to detect and treat, i.e. inhibit the growth of, a variety of malignancies, in addition to other conditions involving NK cell-specific receptor-ligand interactions. Examples of NK-susceptible cancers suitable for detection and treatment through the use of the present invention include, but are not limited to, leukemias and lymphomas. NK-resistant cancers may also be treated if surface expression of p38.5 is increased thereby rendering the cancers NK susceptible. Other conditions involving NK cell-specific receptor-ligand interactions include organ and tissue transplantation (including bone marrow), infectious diseases and autoimmune diseases.

The proteins and functional analogs may also be used to produce antibodies for use as probes to detect the presence of the p38.5 protein or the p70 protein in a sample. The antibodies may be polyclonal or monoclonal. For this purpose, functional analogs include fragments and substitution, addition and deletion mutations of the protein as long as the analogs also produce antibodies capable of detecting the presence of the p38.5 protein or the p70 protein in a sample. The sample may, for example, be a bodily fluid from a mammal, including a human, suspected of harboring an NK-susceptible tumor or NK cells.

The disclosure enables the artisan to use antibodies directed to non-homologous regions of the p38.5 protein or the p70 protein to distinguish cells expressing one of the proteins from cells that express the other protein. Alternatively, an antibody may be employed that has binding affinity for a region that is homologous between the two proteins. In this scenario, the method permits detection, isolation, or quantification of the total number of cells expressing one or both or the proteins as a measure of total gene expression irrespective of variances in splicing or other processing. Of the two forms of the gene that appear to be expressed, one form, the p70 protein, appears to be expressed normally in NK cell, whereas expression of the other form, the p38.5 protein, appears to be abnormal inasmuch as it has heretofore only been identified in tumor cells.

Also described is a method of treating cancers or immune reaction conditions that involve the protein-protein interaction described herein. For example, a mutant form of either the p38.5 protein or the p70 protein (or a fragment of one of the proteins) can be used to inactivate or interfere with the function normally associated with binding of these proteins. Alternatively, nucleic acids encoding one of the proteins or a fragment thereof may be used to induce production of a mutant form that interferes with function. Thus, cells may be transfected with mutant receptor form of protein where the mutant molecule still has ligand-binding capacity, but has an altered functional part thereby preventing intracellular signaling. In this case, the method induces a competitive inhibition whereby the normal ligand is prevented from binding to its receptor, and the normal function is inhibited. For example, this method can be used to down-regulate NK cell function.

Also described is a method for killing tumor cells, comprising administering to an individual having a cancer a cytotoxic agent attached to the tumor cell surface protein p38.5 or the natural killer cell surface protein p70. In this embodiment, the cytotoxic agent can be selected from radioactive agents such as ¹³¹I, protein toxins such as ricin A chain, alkylating agents such as chlorambucil and doxorubicin, antibiotics, e.g., DNA-binding antibiotics such as daunomycin, heavy metal complexes such as cisplatin, and anti-metabolites such as 5-fluorouracil and vinca alkaloids.

Also described is a method for treating cancers comprising inducing the expression of p38.5 cell surface protein by cancer cells and thereby causing the cancer cells to be susceptible to attack by the immune system. For example, target tumor cells that are non-NK-susceptible may be rendered NK-susceptible by transecting the cells with a recombinant expression vector capable of expressing p38.5 on the surface of the cells.

Also described is a method for inhibiting autoimmune reaction in a patient susceptible thereto, comprising interfering with natural killer (NK) cell function by inhibiting p70 expression or activity. For example, the method can be used to inhibit NK function by administering to the patient an antibody that specifically binds to p70 cell surface protein expressed by natural killer cells, thereby inhibiting NK cell activity. Alternatively the method can be used to inhibit NK cell function by administering to the patient a nucleic acid that specifically hybridizes to SEQ ID NO:4, thereby inhibiting expression of p70 cell surface protein expressed by NK cells. Autoimmune conditions amenable to treatment according to the method include, for example, allograft rejection, xenograft rejection, and graft versus host disease.

Also described is a method of distinguishing cells that express p38.5 tumor cell surface protein from cells that express p70 NK cell surface protein. In this approach, the method comprises contacting a biological sample that contains p38.5-expressing cells and p70 expressing cells with p38.5-specific and/or p70-specific nucleic acid probes or primers, such that the probes hybridize to non-homologous regions of one or both of the p38.5 and p70 cDNA sequences, and identifying the bound nucleic acid probe. Cells expressing p38.5 or p70 can be identified and differentiated from one another using conventional analytical techniques, such as Northern analysis, Southern analysis, RT-PCR analysis, flow cytometry, and fluorescent *in situ* hybridization.

In the present study several strategies were employed to over come the difficulties of heterogeneity and the low frequency ofNK cell in HPBL preparations. Only freshly isolated (i.e., non-cultured) NK cells were utilized. Cells from individuals that demonstrated activity against NK resistant-LAK sensitive tumor cell lines were not utilized since the activated NK cells in these fresh preparations might mask the detection of naive NK cell specific ligands. Finally a tagged-ligand cell adsorption technique (see Das et al., J. Exp. Med. 180: 273-281 (1994)), was utilized with enriched NK cell preparations to enhance the likelihood of detection of tumor proteins that selectively bound to the naive killer cells. Using the above approaches and additional biochemical and immunologic techniques we demonstrate for the first time a novel 38.5 kD protein on the plasma membrane of certain tumor cell lines that preferentially reacts with a surface component of naive human NK cells. The interaction appears to be unique to NK cells since T lymphocytes did not bind p38.5. Binding studies have not however been conducted with B cells, monocytes or polymorphonuclear leukocytes.

In addition, a 70 kD protein on the plasma membrane ofNK cells was identified as a p38.5 binding molecule. Consistent with p38.5 cellular binding studies a 70 kD receptor was not detected on T lymphocytes. Addition of purified p38.5 to fresh lymphocyte preparations prior to incubation with K562 target cells substantially decreased NK cell lytic activity. Preferential interaction of p38.5 with NK cells and its blocking activity in functional assays are properties consistent with a role in an early recognition event in NK cell mediated tumor cytolysis.

Additional evidence of a role for p38.5 as a target ligand in naive NK cell mediated cytotoxicity is provided by data demonstrating an association of the expression of this molecule and susceptibility to cytolysis of different tumor cell lines. Flow cytometry and immunoprecipitation studies (of surface labeled cells) revealed that p38.5 is expressed on NK susceptible targets such as K562, MOLT-4 and Jurkat, whereas this molecule was not detected on the plasma membrane of NK resistant, LAK sensitive, targets such as Raji, A549 and MDA-MB-231 suggesting that p38.5 is not involved in LAK mediated cytotoxicity. The functional role of p38.5 in NK cell mediated cytolysis was also demonstrated in studies of p38.5 loss variants. Following long term culture of wild type NK sensitive Jurkat and Molt-4 cell lines, variants were isolated that exhibited decreased levels of p38.5 and reduced susceptibility to lysis by NK cells. This property was not due to a phenotypic alteration in the cells as a result of culture conditions since resistant clones were obtained at limiting dilution (Norin, unpublished data). These studies clearly establish a strong association between the expression of p38.5 on the tumor plasma membrane and susceptibility to NK cell mediated cytolysis.

The following examples are provided to assist in a further understanding of the invention. The particular materials and conditions employed are intended to be further illustrative of the invention and are not limiting upon the reasonable scope thereof.

### EXAMPLES

### Materials

### Chemicals, Antibodies and Cell Lines

N-hydroxy succinamide ester of biotin (biotin-NHS) was purchased from Calbiochem-Novabiochem Corp. (La Jolla, CA). Streptavidin alkaline phosphatase and all cell culture media and reagents were from Gibco BRL (Gaithersburg, MD). Electrophoresis reagents and chemicals were purchased from Bio-Rad Laboratories (Melville, NY). All other chemicals used were from Sigma Chemical Co. (St. Louis, MO).

Mouse anti-rabbit IgG was purchased from Pierce (Rockford, IL), goat anti-rabbit alkaline phosphatase was obtained from Sigma Chemical Co. (St. Louis, MO). Goat anti-rabbit IgG conjugated to FITC was obtained from Tago Inc. (Burlingame, CA). Antibody recognizing p38.5 was raised in a rabbit against an 11-mer synthetic peptide derived from the amino acid sequence of an internal peptide of the molecule (see below). The synthetic peptide (250 µg) was mixed with Titermax (CytRx Co. Atlanta, GA) (1:1 v/v) and injected subcutaneously into a rabbit at 4 sites as instructed by the manufacturer. Subcutaneous booster injections (100 µg peptide in Freund's adjuvant incomplete) were given to the rabbit at bimonthly intervals. Serum was collected after one week following the third booster injection. Anti-p38.5 antibodies were affinity purified from sera using BSA-11 mer peptide conjugate linked to CNBr-Sepharose 4B. Affinity purified antibody had a titer of 1:2,000 and reacted with a single protein of 38.5 kD on Western blots.

Cell lines used in this study were obtained from the American Type Culture Collection (Rockville, MD) and were maintained in media according to instructions provided.

### Example 1: Lymphocytes

Human peripheral blood lymphocytes (HPBL) were isolated as described in Das et al., J. Exp. Med. 180: 273-281 (1994). Blood obtained from healthy volunteers was diluted 1:1 with HBSS, layered on Ficoll-Hypaque® (Pharmacia, Piscataway, NJ) and centrifuged at 200 g for 20 min. The mononuclear leukocyte fraction (Ficoll-Hypaque® interface) was passed through nylon wool columns to obtain HPBL depleted of B cells and monocytes.

### Example 2: Isolation of Naive NK Cells and T Lymphocytes

Naive human NK cells are defined as freshly isolated CD3⁻, CD5⁻, CD16⁺ that exhibit limited target cell specificity, i.e., lytic activity against K562 but not A549 tumor cells. Activated NK cells, i.e., those which are stimulated with lymphokines such as IL-2 (lymphokine activate killer cells, LAK), are able to lyse NK resistant tumor cells such as A549. See Trinchieri (1989) and Whiteside (1996). Only those preparations of HPBL that lacked LAK cell activity were used for the isolation of naive NK cells and T lymphocytes. Isolation of naive NK cells and T lymphocytes was performed as follows. Freshly isolated HPBL were suspended in phosphate buffered saline (PBS) containing 2% fetal bovine serum (FBS) at 20 x 10⁶ cells/ml and incubated (4°C for 30 min) with anti-CD5, 20 µl/1 x 10⁶ cells (Becton-Dickinson, San Jose, CA). Anti-CD5 reactive lymphocytes were captured by incubating the mixture with sheep anti-mouse IgG-coated magnetic beads, 30 µl/1 x 10⁶ cells (Dynal Inc., Lake Success, NY). The magnetic beads containing adherent CD5⁺ cells were washed, suspended in RPMI supplemented with 15% FBS and incubated overnight at 37°C in 5% CO₂. Subsequently CD-enriched lymphocytes (T lymphocytes) were separated from the magnetic beads by vortexing for 2 minutes and placing the mixture in a magnetic field. The supernatant containing CD5⁻ HPBL was incubated with anti-CD3, 20 µl/1 x 10⁶ cells (Becton Dickinson, San Jose, CA) at 4°C for 30 minutes. Anti-CD3-reactive lymphocytes were separated using magnetic beads as described above. The supernatant was centrifuged to obtain cells that were depleted of CD3⁺ and CD5⁺ lymphocytes (i.e., CD 16⁺ enriched naive NK cells). The average phenotype of the T lymphocyte enriched fraction was <3% CD16⁺, 50% CD4⁺ and 93% CB3⁺. These cells did not exhibit NK or LAK cytolytic activity. The average phenotype of the negatively selected naive NK cell enriched fraction was 85% CD16⁺, <3% CD3⁺. These cells exhibited increased NK cell lytic activity (60% cytotoxicity at an effector to target E:T ratios of 100: 1) compared to unfractionated HPBL (32% cytotoxicity).

### Example 3: Binding of Tumor Plasma Membrane Proteins to Lymphocytes

Plasma membrane proteins of viable tumor cells were labeled with biotin as described Das et al. (1994). Briefly, cells (>99% viable by trypan blue dye exclusion) were washed three times with a solution containing 10 mM HEPES, 145 mM NaCl, 4 mM KCl, 11 mM glucose, pH 8.0 (buffer A) and then incubated in the same buffer with biotin-NHS (1 mM biotin-NHS/10x10⁶ cells) for one hour at 4°C. Cells were then washed three times with 20 vol of buffer A to remove unreacted biotin. The biotinylated cells were suspended in 250 mM sucrose with 10 mM HEPES (pH 8.0) and disrupted by N₂ cavitation (1000 psi) in a Parr's chamber. The resulting suspension was centrifuged at 200 g for 5 minutes to remove undisrupted cells and the supernatant was then centrifuged at 30,000 g for 20 minutes to obtain a crude membrane fraction. Membranes were washed once with 10 mM sodium borate. 10 mM benzamidine, 1 mM ethylenediamine tetraacetic acid (EDTA), 1 mM iodoacetamide, 1 mM phenyl methyl sulfonyl fluoride (PMSF), pH 8.0 (buffer B) and incubated in the same solution with 1% Triton X-100 at 4°C overnight. Solubilized membrane proteins were obtained as the 30,000 g supernatant of the detergent treated membranes and dialyzed extensively against buffer B containing 0.05% Triton X-100, as described in Das et al. (1994).

Biotin labeled solubilized tumor plasma membrane proteins were reacted with freshly isolated naive NK cells and T lymphocytes at a ratio of 2:1 (on the basis of cell numbers) in RPMI-1640 supplemented with 15% FBS at 4°C for 2 h. The reacted cells were washed twice with media and three times with buffer A. Finally, cells were solubilized in Laemmli sample buffer and the solubilized proteins were subjected to SDS-PAGE and Western blotting. Biotinylated tumor membrane proteins that bound to lymphocytes were identified with a streptavidin biotin detection system as described in Das et al. (1994).

### Examples 4: Purification of Tumor Membrane Proteins

Membrane proteins were purified as described Das et al. (1994). Approximately 1 x 10¹¹ cells of the erythroleukemia cell line (K562) were washed three times with PBS and used to prepare plasma membrane proteins as described above. Solubilized membrane proteins were extensively dialyzed against buffer B and subjected to preparative SDS-PAGE. A vertical portion of the gel was sliced and stained with Coomassie blue to locate desired proteins. Protein bands of interest were cut from the gel, eluted and further purified by SDS-PAGE. A portion of the purified protein was extensively dialyzed against 10mM Tris-HCl, pH 8.0 and used for cytotoxicity inhibition studies or dialyzed against Buffer B and labeled with biotin as described above for study of its binding properties to various lymphocyte subsets. A portion of the purified protein was also isolated on nitrocellulose paper by SDS-PAGE and transblotted using the method described by Aebersold et al. Proc. Natl. Acad. Sci 84: 6970-6974 (1987) for the purpose of internal amino acid sequence analysis of the protein subsequent to *in situ* protease digestion (Harvard Micro Chemistry Laboratory, Cambridge, MA).

More generally the preparation of tumor or NK cell Plasma Membrane Proteins was carried out as follows. Tumor cells ( >99% viable by trypan blue dye mM NaCl, 4 mM KCl, 11 mM glucose, pH 8.0 (buffer A). Cells were suspended in 250 mM sucrose with 10 mM HEPES (pH 8.0) and disrupted by N₂ cavitation (1000 psi) in a Parr's chamber for 45 minutes. The resulting suspension was centrifuged at 200 g for 5 minutes to remove undisrupted cells and the supernatant was then centrifuged at 30,000 g for 20 minutes to obtain a crude membrane fraction. Membranes were washed once with 10 mM sodium borate, 10 mM benzamidine, 1 mM ethylenediamine tetraacetic acid (EDTA), 1 mM iodoacetamide, 1 mM phenyl methyl sulfonyl fluoride (PMSF), pH 8.0 (buffer B) and incubated in the same solution with 1% Triton X-100 at 4°C overnight. Solubilized membrane proteins were obtained as the 30,000 g supernatant of the detergent treated membranes and dialyzed extensively against buffer B containing 0.05% Triton X-100. This procedure resulted in proteins from the membrane that were relatively water soluble.

Tumor membrane proteins were than purified as follows. Solubilized membrane proteins, as prepared above were incubated with Laemmli's sample buffer at 37 °C for 15 minutes and then subjected to separation based on their molecular weight by preparative SDS-PAGE. A vertical portion of the gel was sliced and stained with Coomassie blue to locate desired proteins. Protein bands of interest were cut from the gel, eluted and further purified by SDS-PAGE in a similar manner. Finally the protein band of interest (i.e, p38.5 or p70) was eluted and dialyzed against a buffer of choice. The purity of the protein was established by subjecting the eluted protein band to gel electrofocusing. Purified protein was thus obtained by electroelution of the band from the second SDS-PAGE or from electrofocusing gel.

Purified proteins, after extensively dialysis against an appropriate physiologic buffer, can be used for production of polyclonal antibodies and/or monoclonal antibodies. The p70 and p38.5 proteins were used to alter the function of NK cells by direct binding to tumor cells and/or NK cells, respectively.

### Example 5: Immunoprecipitation of Surface-Labeled Proteins

incubating anti-p38.5 with anti-rabbit IgG coupled to CNBr- Sepharose 4B at 4°C for 90 min in PBS containing 1% BSA. See Das et al. (1994). The reacted beads were then washed three times with buffer and incubated at 4°C for 90 minutes with surface biotinylated tumor plasma membrane proteins. Beads were then washed three times with PBS containing 1% BSA and two times with PBS alone, suspended in Laemmli SDS-PAGE sample buffer and boiled for 10 minutes. The supernatant was subjected to SDS-PAGE and proteins transblotted to Immobilon-P membrane (Millipore Corp., Bedford, MA). Anti-p38.5 immunoprecipitated biotinylated tumor membrane proteins were identified on Western blots by reaction with a streptavidin-biotin detection system.

### Example 6: Fluorescence Activated Flow Cytometry

Approximately, 1 x 10⁶ cells were washed three times with PBS containing 0.1 % BSA (PBS-BSA) and incubated with appropriately diluted anti-p38.5 at 4°C for 30 minutes. Cells were then washed three times with PBS-BSA and reacted with FITC-F(ab')₂ goat anti-rabbit IgG for 30 minutes at 4°C. Finally, cells were washed three times with PBS-BSA, resuspended to a concentration of 1 x 10⁶/ ml and analyzed on a FACSort® Flow Cytometer (Becton Dickinson, San Jose, CA). See, for example, Current Protocols in Immunology, Coligan et al., eds., John Wiley & Sons (1991).

### Example 7: Lymphocyte Mediated Cytotoxicity

Cytolysis of tumor cells by lymphocytes was measured as described in Das et al. (1994) and Karpel and Norin, Chest 96:794-798 (1989). Briefly, 100 µl of ⁵¹Cr-labeled target cells (2 x 10⁴ cells/ml) was mixed with 100 µl of lymphocytes (2 x 10⁶ cells/ml) to yield serial two fold dilutions of E/T cell ratios of 100:1 1 to 6:1. Cells were incubated at 37°C for 3 hours in 5% CO₂. Incubation of target cells without the effector cells and in media alone served as a control for the spontaneous release of ⁵¹Cr, and wells without effector cells but with 1% SDS provided the maximum amount of radioactivity present. Cytolysis was calculated as percent Cytotoxicity = 100 x [(CPMₑₓₚ - CPM_{spont.})/(CPMₘₐₓ - CPM_{spont.})].

### Example 8: Binding of Tumor Plasma Membrane Protein p38.5 to Human Lymphocytes

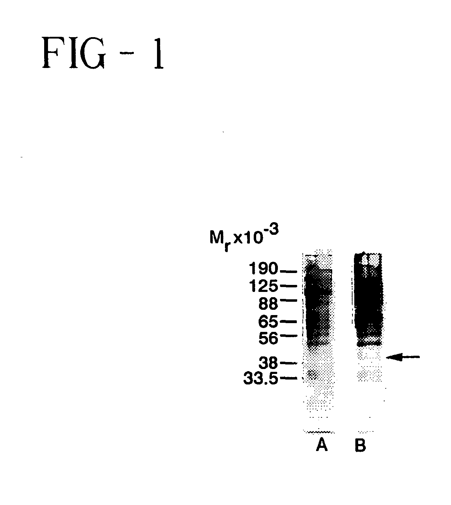

A tagged ligand-cell adsorption technique, see Das et al. (1994), was used to identify membrane proteins from a susceptible tumor cell line that preferentially bind to NK cells. Solubilized membrane proteins from surface biotin-labeled K562 cells were reacted with immunomagnetic bead enriched freshly isolated naive NK cells or T lymphocytes. The membrane proteins from surface biotinylated K562 cells were reacted with freshly isolated viable CD3⁻, CD16⁺ enriched naive NK cells, and CD3⁺, CD16-enriched T lymphocytes. Reacted lymphocytes were washed and solubilized in Laemmli sample buffer. The proteins were then resolved by SDS-PAGE. Lymphocyte bound biotinylated tumor plasma membrane proteins were detected on Western blots with streptavidin-alkaline phosphatase. The results are shown in Figure 1 where lane A is a representative Western blot of surface labeled K562 membrane proteins that bound to enriched T lymphocytes and lane B is a representative Western blot of surface labeled K562 membrane proteins that bound to enriched NK cells. Densitometry analysis of the Western blots, employing Gelbase software (UVP's System 5000, Upland, CA), detected approximately 14 common bands of K562 plasma membrane proteins that bound to both NK cells and T lymphocytes. However, a single band of 38.5 kD (p38.5) was detected from NK cell adsorbed preparations but was not observed in T lymphocyte adsorbed preparations.

### Example 9: Binding of Purified p38.5 to Human Peripheral Blood Lymphocytes

Initial results suggested that p38.5 interacts with a receptor on the surface of naive NK cells which was not expressed on T lymphocytes. Accordingly, p38.5 was purified by preparative SDS-PAGE to apparent homogeneity for additional studies.

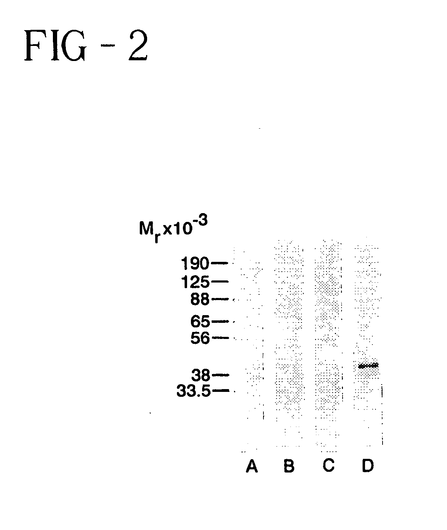

Binding reactions with purified p38.5 and detection of bound biotin labeled p38.5 were performed as described in Example 8. The results are shown in Figure 2. Lane A is a Western blot of Coomassie blue-stained purified p38.5 K562 membrane protein. Lane B is a Western blot of an extract of viable HPBL that were reacted with purified biotin labeled p38.5 and shows slight reactivity. Lane C is a Western blot of an extract of viable enriched T lymphocytes that were reacted with purified biotin-labeled p38.5 and reveals little or no reactivity. Lane D is a Western blot of an extract of viable enriched NK cells that were reacted with purified biotin-labeled p38.5 demonstrating extensive reactivity. As shown in Figure 2, lane A, the purified protein resolved as a single band of 38.5 kD molecular mass upon SDS-PAGE. For examining the binding properties of p38.5 to lymphocyte subsets, p38.5 was labeled with biotin, and the study carried out as described for proteins from the crude membrane preparation.

Figure 2 shows that biotinylated p38.5 bound extensively to the NK cell enriched fraction (see Figure 2, lane D), but did not bind at all to the enriched T lymphocytes (see Figure 2, lane C). Unfractionated cells bound a slight amount of p38.5 (see Figure 2, lane B) which is consistent with the low percentage ofNK cells in peripheral blood lymphocyte specimens. These data also demonstrated that SDS-PAGE purified p38.5 retained its preferential binding property for naive NK cells.

### Example 10: Structural Characteristics of p38.5

Initial attempts to obtain a partial amino acid sequence of the purified p38.5 were unsuccessful because the N-terminus of the molecule was blocked. Consequently, p38.5 was transferred onto nitrocellulose paper and subjected to insitu protease digestion as described in Aebersold et al., Proc. Natl. Acad Sci 84: 6970-6974 (1987). Amino acid sequence analysis of resultant peptides was performed at the Harvard Microchemistry Laboratory, Cambridge, MA. An 11-mer amino acid sequence of an internal peptide was obtained with a high degree of confidence (SEQ ID NO:3). Comparison of this sequence with known sequences of proteins in Gen-Bank did not reveal significant homology. Treatment of p38.5 with sulfhydryl reducing agents (2-mercaptoethanol and dithiothreitol) or deglycosylating enzymes (O-glycosidase, N-acetylneuraminidase II and peptide-N-glycosidase F, deglycosylation Kit, Glyko Inc., Novato, CA) did not alter the molecular mass of the molecule. This suggests that the isolated protein is a monomer and may not be glycosylated.

### Example 11: Expression of p38.5 on Established Tumor Cell Lines

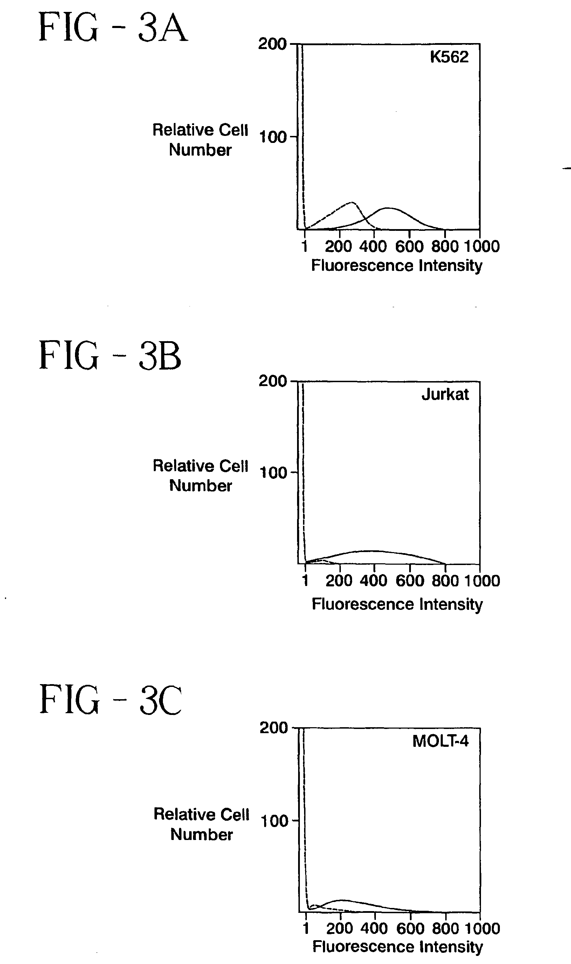

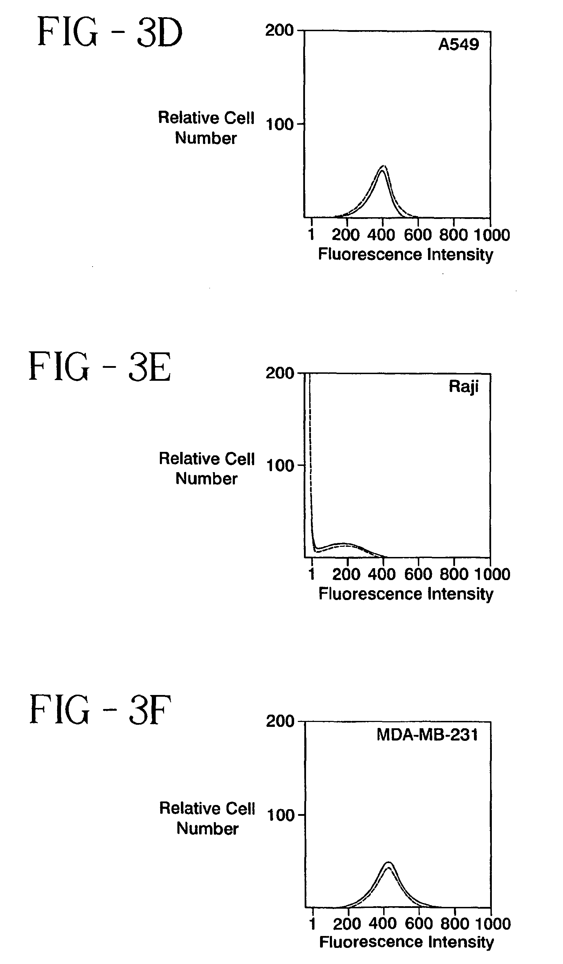

Since p38.5 (derived from K562 cells) exhibited selective binding to naive NK cells, it was of interest to examine the expression of this molecule on the plasma membrane of NK resistant cell lines as well as other NK sensitive tumor cell lines. Affinity purified antibody raised against the internal 11 mer synthetic peptide of p38.5 (anti-p38.5) was employed for this purpose. Anti-p38.5 was reacted with three NK sensitive and three NK resistant tumor cell lines and analyzed separately by flow cytometry. The flow cytometry analysis of anti-p38.5 treated NK-susceptible and NK-resistant tumor cell lines is shown in Figure 3A to Figure 3F. Cells were treated with anti-p38.5 followed by goat anti-rabbit F(ab')₂ IgG - FITC (_). Control tumor cells were treated with secondary antibody alone (----). Non-immune rabbit serum had similar reactivity as secondary antibody alone (data not shown). As shown in Figure 3A to Figure 3F, anti-p38.5 reacted with the surface of NK sensitive tumor cell lines, K562 (erythroleukemia), Jurkat (T cell lymphoma) and Molt-4 (T cell leukemia). In contrast, anti-p38.5 did not bind to the three NK resistant tumor cell lines A549 (lung adenocarcinoma), Raji (Burkitt lymphoma) and MDA - MB231 (breast carcinoma).

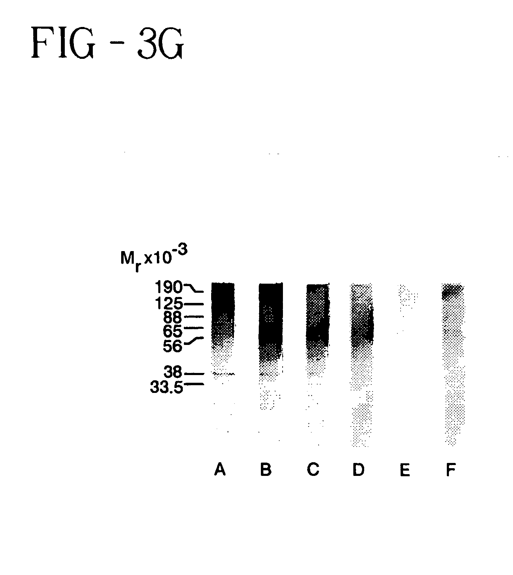

Plasma membrane expression of p38.5 was also studied by anti-p38.5 immunoprecipitation of surface labeled (biotin) membrane proteins of the above tumor cells. Figure 3G shows Western blots of anti-p38.5 immunoprecipitated proteins from surface biotinylated cells: K562 (lane A), Jurkat (lane B), Molt-4 (lane C), Raji, (lane D), A549 (lane E), and MDA-MB 231 (lane F). Proteins in these lanes were probed with streptavidin-alkaline phosphatase to detect cell surface biotinylated proteins. Biotin labeling and immunoprecipitation of plasma membrane proteins is described, for example, in Das et al. (1994). Analysis of the Western blots showed the presence of a single 38.5 kD biotinylated protein from NK-sensitive cell lines, whereas biotinylated proteins were not observed when the NK-resistant cell lines were employed. These results demonstrated an association between the expression of p38.5 on the surface of tumor cell lines and their susceptibility to NK cell-mediated lysis.

### Example 12: Cell Surface Expression of p38.5 on NK Resistant Variants of Jurkat and Molt-4 Cell Lines

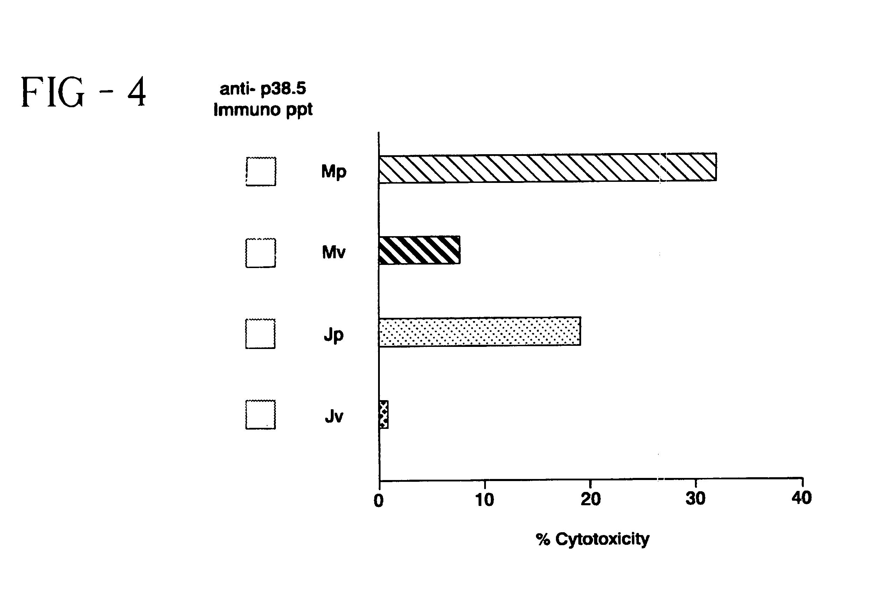

Though the expression of p38.5 is associated with susceptibility of well-established tumor cell lines to NK cell mediated cytolysis, one must interpret such data with caution as the cell lines are derived from different tissues and are likely to have been transformed by different mechanisms. This issue was addressed by examining the association of NK susceptibility and p38.5 expression in variants derived from wild type tumor cells. Following long term culture of Jurkat and Molt-4 cell lines, a substantial reduction in their susceptibility to NK cell-mediated cytotoxicity was observed. These variant cell lines were then analyzed for surface expression of p38.5 by anti-p38.5 immunoprecipitation. These results are shown in Figure 4. Parental cell lines = Mp, Jp; Variant cell lines = Mv, Jv. Following long term culture of Jurkat (J) and Molt-4 (M) cell lines, substantial reduction in NK cell-mediated cytolysis was observed. These variant cell lines were analyze d for surface expression of p38.5 using a specific antibody as described above. Membrane proteins derived from 1x10⁶ cells were loaded in each lane. The level of cytolytic activity was directly related to the apparent degree of expression of surface p38.5. Gelbase analysis (UVP, Upland CA) of the blots for protein bands from parental cell lines demonstrated relative single peak heights of 28 and 25, respectively. Similar analysis of the blots for protein bands from the variant cell lines did not reveal anti-p38.5 reactive protein. However, a very light band was visible by the naked eye (for Mv). The results shown in Figure 4 demonstrated that variant cell lines exhibit decreased susceptibility to lysis by NK cells and express significantly lower amounts of p38.5 on their surface as compared to the wild type parental cell lines.

### Example 13: Effect of Purified p38.5 on NK Cell Mediated Cytolysis

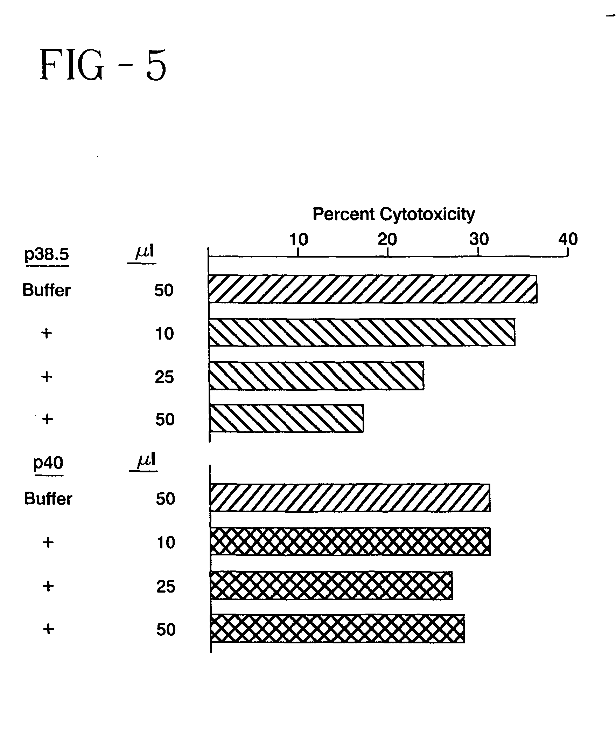

The preferential binding of p38.5 to NK cells suggested that the molecule may be involved as a ligand in the cytolytic process mediated by naive NK cells. To directly examine this possibility, a purified preparation of p38.5 was incubated with human lymphocytes and then tested in a standard cytotoxicity assay against ⁵¹Cr-labeled K562 target cells. Incubation of lymphocytes with the purified p38.5 preparation inhibited naive NK activity in a concentration dependent manner (see Figure 5). Human peripheral blood lymphocytes (HPBL) were pre-incubated with the indicated amount of purified proteins (approximately 45 ng/µl based on amino acid sequence data) at 37°C for 30 minutes and then added to ⁵¹Cr-labeled K562 cells at an E/T ratio of 100:1 1 as previously described. Percent cytotoxicity was determined after 3 hours incubation by the release of ⁵¹Cr. See Das et al. (1994); Karpel and Norin (1989). Representative data of two experiments are shown in figure 5. The cytotoxicity data were analyzed by the Student's t test. The results obtained were p38.5, 10µl, not significant; p38.5, 25µl, p<0.01; p38.5, 50µl, p<0.001 compared to the buffer control. p40 values compared to buffer control were not significant at any concentration.

These data provide direct evidence that interaction of the tumor surface protein p38.5 with its NK cell receptor is necessary for cytotoxicity since binding of soluble ligand to the NK cells prior to contact with K562 cells inhibited their lysis. A K562 membrane protein of 40 kD, purified by the same procedure as p38.5, did not affect NK activity.

### Example 14: Characterization of NK Cell Receptor of p38.5

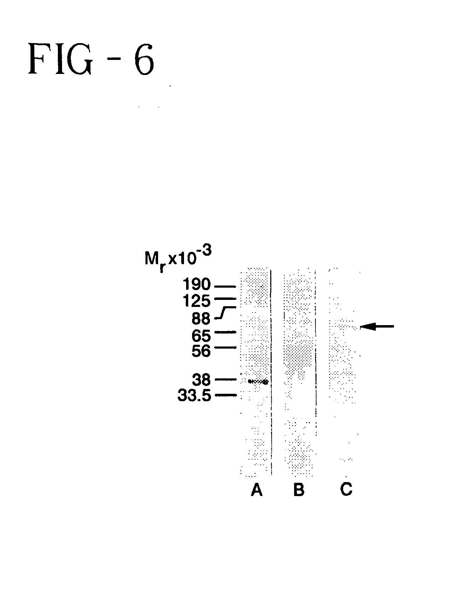

In order to further delineate the preferential interaction of p38.5 with NK cells relative to T lymphocytes, we conducted investigations to identify an interactive molecular species on effector cells. In direct binding assays, biotin labeled purified p38.5 was reacted with either NK cell or T lymphocyte membrane proteins which had been previously immobilized on Western blots. The results are shown in Figure 6. Lane A is a Western blot of Coomassie blue stained purified p38.5 K562 membrane protein. Lane B is a Western blot of T lymphocyte proteins that were reacted with purified biotinylated p38.5. (Bound p38.5 was not detected on this blot indicating the absence of reactive T lymphocyte proteins.) Lane C is a Western blot of NK cell proteins that were reacted with purified biotinylated p38.5. Labeled p38.5 was detected at approximately 70 kD demonstrating a preferential interaction with the latter immobilized NK cell protein. The reactivity of p38.5 with the 70 kD NK cell protein was markedly reduced when an extensively denatured (by boiling) preparation of NK cell proteins was employed (data not shown). As shown in Figure 6, labeled p38.5 was detected as a single band at 70 kD on blots of NK cell membrane proteins, whereas reactive bands were not detected on blots of T lymphocyte membrane proteins. This result suggests that a protein of 70 kD from naive NK cells binds cell surface p38.5 from NK cell-susceptible tumors and that the 70 kD protein is either not present or is present in substantially lower amounts in T lymphocytes.

### Example 15: Immunologic Identification of p38.5 bound to p70

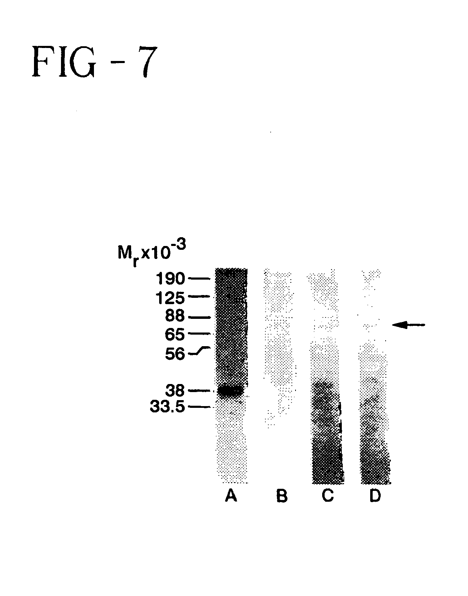

To establish the immunologic identity of the ligand bound to the NK cell receptor, anti-p38.5 serum was employed. A Western blot of peripheral blood lymphocyte membrane proteins was initially incubated with a solubilized crude extract of K562 membrane proteins, washed and then reacted with anti-p38.5. These results are shown in Figure 7. Lane A is a Western blot of K562 membrane protein extract that was reacted with affinity purified anti-p38.5. Lane B is a Western blot of HPBL membrane proteins reacted with affinity purified anti-p38.5. Note the absence of anti-p38.5 reactive proteins. Lane C is a Western blot of HPBL membrane proteins reacted with K562 membrane extracts for 2 h at ambient temperature, washed and probed with anti-p38.5. Note the detection of a band at an approximate molecular weight of 70 kD. Lane D is a Western blot, same as Lane C, but with twice the amount of K562 membrane proteins. All lanes were developed with goat anti-rabbit alkaline phosphatase.

From Figure 7, it can be seen that the antiserum reacted with a band at 70 kD rather than at 38.5 kD (compare lanes C and D to lane A). Western blots of lymphocyte membrane proteins that were not pre-incubated with the tumor proteins did not react with anti-p38.5 (see lane B). These results suggest that the 38.5 kD surface molecule from K562 cells which binds to the 70 kD naive NK cell protein contains the 11 mer peptide to which antibody was raised. It can be concluded from these results that the 11-mer epitope of p38.5 is probably not directly involved in the receptor-ligand interaction. (Presumably if the p38.5-70 kD interaction included the 11 mer epitope of p38.5, antibody to the latter determinant could not bind to the complex).

### Example 16: Plasma Membrane Localization of p70

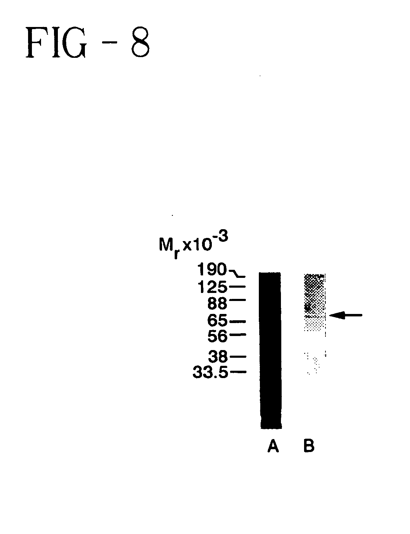

The following experiment was performed to confirm that a 70 kD NK cell protein (p70) that specifically binds p38.5 is localized on the plasma membrane. Solubilized membrane proteins from surface biotinylated naive NK cells were applied to a p38.5-Sepharose-4B affinity column. After extensive washing with 0.5 M NaCl buffer to remove non-specifically bound NK cell membrane proteins, the remaining specifically bound proteins were eluted by solubilization of the beads with Laemmli sample buffer (denaturing buffer), resolved by SDS-PAGE, and subjected to Western blot analysis. p38.5-Sepharose 4B bound NK cell membrane proteins were detected on Western blots using standard streptavidin-alkaline phosphatase reaction. These results are shown in Figure 8. Lane A shows crude extract of NK cell membrane proteins from surface biotinylated NK cells prior to affinity chromatography. Lane B shows cell membrane protein(s) specifically bound to the p38.5 affinity column. A single discrete biotinylated band of 70 kD from NK cells was detected on the blots suggesting that this p38.5 binding protein (p70) is located on the exterior of the plasma membrane (the cell surface). See Figure 8, lane B. One possibility is that the 70 kD, p38.5 binding protein is the inducible form of heat shock protein (Hsp 70). We performed flow cytometry studies utilizing antibody to Hsp 70 (Stressgen Biotech. Corp. Victoria, BC Canada, Clone C92F3A-5) to determine surface expression of this molecule on T and NK cells. These experiments indicated that neither NK nor T cells expressed Hsp70 on their plasma membranes in contrast to K562 cells which served as a positive control (Norin, unpublished data).

### Example 17: Construction of NK cell cDNA library

Construction of cDNA library of highly enriched naive NK cells was achieved using the following methods.

### a. Isolation of NK Cells

Human peripheral blood lymphocytes (HPBL) were fractionated to enrich for NK cells. Non-adherent HPBL were obtained after overnight incubation at 4°C on a plastic disk. These non-adherent HPBL were subjected to two cycles of nylon wool column adsorption to obtain lymphoctes further depleted of B cells and monocytes. These lymphocytes were then treated with anti-HLA class II immunomagnetic beads. Negative cells (i.e., those that did not bind to the beads) were treated with anti-CD5 and anti- CD3 (Becton-Dickenson, San Jose, CA) in succession to remove T lymphocytes utilizing goat anti-mouse IgG immunomagnetic beads (Dynal Inc., Lake Success, NY). This negatively selected lymphocyte preparation (class II⁻, CD3⁻, and CD5⁻, and enriched for CD16⁺ NK cells) was utilized for construction of the cDNA library. The positively selected T cells were used for RNA subtraction.

### b. Purification of NK cell mRNA by subtraction hybridization.

mRNA from naive NK cells (HLA class II⁻, CD3⁻ CD5⁻, CD16⁺) was isolated utilizing an mRNA isolation kit (Stratagene, La Jolla, CA). NK cell mRNA was then utilized to make single strand cDNA (Stratagene cDNA library kit), whereas T cell mRNA was photobiotinylated following instructions provided in the subtraction kit (Invitrogen, San Diego, CA). NK cDNA and T cell biotinylated mRNA were hybridized (for 36 hours) and the hybridization mixture was treated with streptavidin and extracted to remove hybridized cDNA-mRNA and free mRNA. See the Invitrogen subtraction kit manual for further details. Hybridization was repeated once more to ensure complete removal of contaminating biotinylated hybridized T cell mRNA and free mRNA. Unique NK cell cDNA (in aqueous phase) was further processed as described below.

### c. Preparation of cDNA library.

Subtracted NK cell cDNA obtained in the procedure described in Example 17b was utilized to clone in Lambda ZaP Express using the Gigapack III Gold Cloning kit according to the instruction manual (Stratagene, La Jolla, CA). Briefly, single strand NK cell cDNA was utilized to synthesize the second strand. The termini of the double-stranded cDNA were filled in with Pfu DNA polymerase and EcoR I adapters were ligated to the blunt ends. Subsequently, EcoRI adapter and residual linker-primer from the 3' end of the cDNA were released by Xho 1 digestion. Fragments were then separated on a Sephacryl S 500 spin column. The size fractionated cDNA was then precipitated and ligated to the Lambda Zap Express vector arms. The Lambda library was packaged in a high-efficiency system of Gigapack III Gold packaging extract and finally the library was plated on the *E.Coli* cell line XL1 - Blue MRF'.

### d. Screening of the library for NK Cell p70.

The strategy for isolation of clones that express p70 was to identify those plaques which bind p38.5 and therefore acquire reactivity for anti-p38.5 antibodies. Plaques of XL1-Blue MRF' infected with λ phage were transferred to nitrocellulose paper. After appropriate blocking with 5% BSA, the paper was initially treated with the solubilized extract of K562 membrane protein (containing the p38.5 ligand) washed and then incubated with anti-p38.5 antibodies. Anti-p38.5 reactive plaques were identified by reaction with biotinylated goat anti-rabbit IgG followed by streptavidin alkaline phosphatase and NBT/BCIP mixture. Positive plaques were purified further by secondary and tertiary screening. Positive reaction of plaques without incubation with K562 membrane protein served as a control.

### e. Sequencing of positive inserts.

Purified positive plaques were used in an in vivo excision protocols utilizing Exassist helper phage and XLOLR *E*. *coli* strain. This procedure enables one to obtain colonies containing a PBK-CMV double-stranded phagemid vector with the cloned DNA insert (details of the procedure are given in the Strategene Instruction manual). XLOLR strain of such phagemid vector were then utilized to isolate the plasmid using a plasmid isolation kit supplied by Qiagen (Chatsworth, CA). This plasmid with appropriate primers was used to obtain the oligonuclotide sequence of the insert. The nucleic acid sequencing was performed by Commonwealth Biotech Inc. (Richmond, VA) and SUNY Health Science Center Brooklyn Nucleic Acid Sequencing Lab on Nucleic Acid Sequencer 377 ABI X-L and 373 ABI (Perkin Elmer Applied Biosystems Inc.), respectively. A clone was obtained that encodes a p70 cDNA fragment of 1.315Kb and contains an open reading frame of 254 amino acids. The remaining sequence of the cDNA of the p70 NK receptor will be obtained by primer extension using 3' gene specific nested primers from the initial fragment and a 5' primer from T3 or by using the 5' RACE® kit of GIBCO-BRL (Grand Island, NY) or by rescreening the library with antibodies and/or oligonucleotide probes based on the initial sequence.

### Example 18: Isolation and seqencing of the cDNA for the p38.5 tumor protein

A commercially available K562 cDNA expression library (Clontech) in Lambda gt11 was screened with antibodies to p38.5. Positive clones were confirmed by Southern analysis using a degenerate probe from the 11-mer peptide. A positive clone was sequenced by Commonwealth Biotech Inc. (Richmond, VA.) and SUNY Health Science Center Brooklyn Nucleic Acid Sequencing Lab on Nucleic Acid Sequencer 377 ABI X-L and 373 ABI (Perkin Elmer Applied Biosystems Inc.), respectively. A fragment of the cDNA of 730 nucleotides encoding 243 amino acids was obtained. The remaining sequence will be obtained by primer extension using the 3' and 5' RACE kits of (GIBCO-BRL) or by selecting additional clones for sequencing from a Lambda based library.

### Example 19: DNA analysis

Total cellular DNA for Southern analysis were prepared by proteinase K digestion and subsequent phenol/chloroform extraction and ethanol precipitation. RNA was removed by digestion with 50 µg/ml RNase A. From each sample, 25 µg of DNA was digested with EcoRI and electrophoresed on a 0.8% agarose gel. The DNA on the gel were denatured, neutralized and transferred onto nitrocellulose (Schleicher and Schuell, Keene, NH) in 20x SSC overnight. The blot was baked, then blocked in hybridization solution composed of 50% formamide, 200 mM PIPES, 800 mM NaCl, 0.1 % SDS, and 20 mg/ml blocking reagent (Boehringer Mannheim, Indianapolis, IN). Digoxigenin labeled oligonucleotide probes were labeled by random priming (using Prime-it II, Stratagene, LaJolla, CA), added to the hybridization solution and incubated at 42°C overnight. The hybridized blots will be washed at 42°C hybridization temperature in 0.1x SSC/0.1% SDS and developed with anti-digoxigenin alkaline phosphatase.

### Example 20: RNA analysis

Total cellular RNA were prepared by guanidine thiocyanate extraction as described above. Northern blot analysis will be performed by electrophoretic separation of total cellular RNA (15 µg) in denaturing formaldeyde-agarose gels, transfer of the RNA to nitrocellulose membrane (Schleicher and Schull), baking and hybridizing overnight to ³²p random-primed cDNA probes. The hybridized blots will be washed at 60°C in 0.1 x SSC/0.1% SDS and exposed to film for 1-5 days. Reverse Transcriptase-Polymerase Chain Reaction (RT-PCR) has been used and will be used to detect p38.5 and p70 specific mRNA. Two gene specific primers can be used to reverse transcribe mRNA into cDNA and then amplify the cDNA by PCR. RT-PCR is performed with commercial kits such as Titan® (Boehringer Mannheim, Indianapolis, IN)

### Example 21:Transfection of p70 and p38.5 Expression Vectors and Delivery of Antisense Oligonucleotides

Transfection of the p70 cDNA, cDNA fragments thereof, mutant p70 cDNAs, p38.5, and p38.5 mutants employs a pcDNA 3.1/zeo vector (Invitrogen, San Diego, CA) or other suitable vectors, such as Baculovirus based vectors (Novagen, Inc, Madison, WI) according to the manufacturers instructions. The p70 cDNAs or the p38.5 cDNAs or anti-sense cDNAs, respectively, are ligated to the pcDNA 3.1/zeo vector with appropriate restriction enzymes and T4 ligase. The gene-vector construct is amplified in E.coli following transformation and is purified on a plasmid purification column (Qiagen, Valencia, CA). Transfection is accomplished by a liposome mediated technique. An example of a liposome mediate technique for transfection is Lipofectin (Gibco-BRL).

### Example 22: Production of Fusion Protein or Peptides of p70 and p38.5

The pET-32 LIC vector or other suitable vectors such as Baculovirus based vectors (Novagen, Madison, WI) are used according to the manufactures instructions. Full length p38.5 and p70 are used as templates for PCR. Primers are designed according to the Novagen manual and contain a short vector overhang sequence linked by an ATX triplet to a gene specific oligonucleotide. Various fragments of the cDNA or a full length cDNA are cloned into the vector by appropriate selection of primers to produce fusion proteins (peptides of different length). The PCR product is eluted from an agarose gel and ligated to the pET-32 vector. The plasmid is purified by a plasmid isolation kit (Qiagen) and used to transform competent *E*. *coli* supplied in the Novagen kit. Expression of recombinant proteins is by incubation of log phase E.coli with IPTG. The His tagged fusion protein is then purified by affinity on a Ni column. Fusion protein production is verified by SDS-PAGE and Western blot analysis. Fusion proteins/peptides of p70 and p38.5 are used as immunogens for production of polyclonal and monoclonal antibodies.

### Example 23: Shared Regions of Nucleic Acid and Amino Acid Sequence Homology of p38.5 and p70

The cloned cDNA fragment of p38.5 has a region of sequence identity or near identity (starting at nucleotide number 91, codon CAT to the end of the fragment, nucleotide number 730, "G") to the cloned cDNA fragment of p70 (starting at nucleotide number 13, codon CAT to nucleotide number 651, "G"). The identity of nucleotides at positions number 412 and number 416 of the p38.5 cDNA fragment are either G or T and G or A, respectively. The corresponding nucleotide positions in the homologous region of the cDNA fragment of p70 are number 334 and number 338 and the nucleotides at these positions are also either G or T and G or A, respectively. Translation of the resulting alternative codons at these positions results in amino acids Ala/Ser and Arg/His. The cDNA fragment of p70 extends 107 nucleotides beyond the end of the p38.5 cDNA fragment where there is a translation stop codon, TGA, at nucleotide positions 763 to 765. The 3' primers derived from the nucleotide sequence in this 107 base pair region of p70 have been used to reverse transcribe and amplify appropriate size fragments of mRNA from the K562 cell line, a tumor line that expresses p38.5 protein but not p70 protein. This experiment suggests that the region of nucleic acid sequence homology (and consequently amino acid sequence fluorescent activated flow cytometer. Cells that express p38.5 and/or p70 on their surface may be enumerated by this method.

Other methods for identifying p38.5 or p70 proteins using antibody methods include enzyme linked immunosorbant type assays such as enzyme-linked immunosorbent assay (ELISA), Western bloting, and immunoprecipitation. These methods are normally based on incubating an antibody with the sample suspected of containing the protein and detecting the presence of a complex between the antibody and the protein. The antibody is labeled either before, during, or after the incubation step. The protein is preferably immobilized prior to detection. Immobilization may be accomplished by directly binding the protein to a solid surface, such as a microtiter well, or by binding the protein to immobilized antibodies. Immunoassays, such as these, are described by David et al., U.S. Patent No. 4,376,110.

Similarly, nucleic acid probes and primers can be utilized to identify cells that express p38.5 and/or p70 mRNA. This is accomplished by standard Northern, Southern or RT-PCR analyses as described in Current Protocols in Molecular Biology, Ausubel FM et al., eds. John Wiley & Sons (1991).

Antibodies (polyclonal and/or monoclonal) can be developed against non homologous parts of p38.5 and p70 that will specifically identify one or the other of these molecules, respectively, and may alter the function of cells upon binding. A fluorescent label, such as FITC or other suitable fluorescent probes can be conjugated to antibodies directed against non homologous regions of the two molecules which would then bind to cells expressing p38.5 and/or p70 respectively. This method would thereby identify cells expressing either or both of the molecules (by using different labels on each of the different antibodies) by analysis in a fluorescent activated flow cytometer. Cells that express p38.5 and or p70 on may be enumerated by this method. Other methods of identifying p38.5 or p70 proteins using the above antibody methods are enzyme linked immunosorbant types of assays (ELISA), Western bloting and immunoprecipitation. Similarly, nucleic acid probes and primers can be utilized to identify cells that express p38.5 and or p70 mRNA by utilizing their unique and different nucleotide sequences. This is accomplished by standard Northern, Southern or RT-PCR analyses as described above.

### Example 25: Production of Antibodies to p38.5 and p70

Polyclonal antibodies to the 11-mer peptide (SEQ ID NO:3) found in the p38.5 and p70 proteins were produced by inoculation of rabbits, SC, with 250 µg of synthetic peptide in Titermax® adjuvant (CytRX Co., Atlanta, GA) followed by bimonthly (x3) injections of 100 µg of peptide in Freund's incomplete adjuvant. Rabbits were bled and the IgG fracrion of serum was prepared by standard techniques. Antibody titer was established by Western blot analysis.

Polyclonal antibodies to other portions of p38.5 and p70 can be prepared by the method described above, except that the whole protein or fragments of the protein are used.

### Example 26: Preparation of Monoclonal Antibodies

Monoclonal antibodies will be generated by subcutaneous (SC) injection of mice with p38.5 and/or p70 or fragments of these protein molecules. Such immunogens may be generated as fusion proteins/polypeptides, as described in Example 22. The adjuvant and immunization schedule will be similar to that as described for polyclonal antibodies. The clone cell-HY Monoclonal Antibody Production Kit (Stem Cell Technologies, Inc., Vancouver, BC Canada) can be used according to the manufacturers instructions. Spleen cells from immunized mice are isolated and fused to mouse myeloma cells by the polyethylene glycol method. Fused and isolated cells are incubated 16 to 24 hours at 37°C in 5% CO₂ in recovery medium. The cells are then washed and placed into semisolid methylcellulose-HAT-based selection medium and plated into petri dishes. Monoclonal colonies will arise in 10-14 days and are picked up with a pipette and transferred to individual wells in a 96-well tray. The supernatants from the wells are tested for the desired antibody using either ELISA, Western blot, or flow cytometry. The desired hybridomas are expanded in growth medium. Large quantities of high titer antibodies can ve produced by the ascities method. See, for example, Current Protocols in Immunology, Coligan et al., eds., John Wiley & Sons (1991).

### Example 27: Treatment of Autoimmune Reactions that Involve NK Cells

NK cells may play a role in autoimmune reactions, for example diabetis, rheumatoid diseases and multiple sclerosis, and additionally in the rejection of allogeneic and xenogeneic organs, tissues and cells by direct cytolytic interaction with target cells or by the release of cytokines. The activity of NK cell can be altered by interaction with specific antibodies to the p70 protein or by competitive inhibition with soluble ligand (p.38.5). See Das et al. J. Exp. Med. 185:1735 (1997). A patient with the above disease or condition can be injected with antibodies to p70 which may directly destroy NK cells via the action of complement or by macrophages (eg., phagocytosis). Alternatively cytotoxic agents such as ¹³¹I, ricin A chain, chlorambucil and doxorubicin can be conjugated to the anti-p70 antibodies by standard methods and injected into patients with the above conditions. The toxic drug-anti-p70 conjugates will bind specifically to NK cells thus causing their destruction. Toxic drugs can also be conjugated to the ligand of p70 (ie., p38.5 or a fragment thereof) and injected into a patients with the above conditions thus destroying the NK cell upon specific binding.

### Example 28: Treatment of Cancerous Diseases

Therapeutic strategies for treatment of patients with cancerous diseases can be developed. Tumor cells that express p38.5 on their surface can be destroyed by antibodies to this protein. Patients with cancer are injected with appropriate amounts of anti-p38.5 to effect destruction of tumor cells by antibody and complement dependent lysis, antibody dependent cell mediated cytotoxicity or by macrophages (e.g., phagocytosis). Toxin-anti-p38.5 conjugates (similar to the above description) can be injected into patients with cancer. The immunotoxin will bind specifically to tumor cells expressing p38.5 thus effecting their destruction. Toxins conjugated to the ligand of p38.5 (i.e., p70 or a fragment thereof) can be injected into patients. These toxin-p70 conjugates will bind specifically to tumor cells expressing p38.5 thus causing their destruction. Patients may also experience therapeutic benefit following injection of p70 or fragments or analogs thereof followed by injection of antibodies to p70 or p38.5. The antibodies will crosslink the ligand-receptor complexes thus inducing tumor cell apoptosis.

Tumors that do not express p38.5 on their cell surface can be induced to do so by transfection of expression vectors (as described above) that contains appropriate cDNA inserts, e.g., those encoding p38.5. Patients will be injected with appropriate inocula containing the expression vector. Tumor cells can be specifically targeted for transfection using vehicles which bind to the cancer cells (e.g., antibodies to F₁βATPase, Das et al., JExp. Med. 180: 273 (1994). Patients are then injected with antibodies or other reagents directed against the p38.5 protein as described above.

### Example 29: Purification of NK Cells or Tumor Cells by anti-p70 and anti-p38.5 Antibody Coated Magnetic Particle Technology

NK cells comprise less than 10% of the lymphocytes in peripheral blood and in lymphoid tissues. In order to enrich for NK cells, a blood sample or a tissue sample can be incubated with magnetizable particles that have antibodies to p70 fixed to their surfaces. The NK cells (which express cell surface p70) will bind to the particles. The particle-cell mixture is then placed into a tube or column which is subsequently placed in a magnetic field. The magnetized particles (with NK cells attached) will remain attached to the solid support whereas all other cells not attached to the particles can be removed by washing the column or by gentle aspiration. Similarly tumor cells can be separated from normal cells by using particles coated with antibodies that are directed against p38.5. Cells that are enriched by these methods can be used as immunogens, or for further diagnostic tests that require purified cells, or other applications such as purification of biochemicals that are produced by these cells.

### Example 30: NK Cells in Regulation of Reproduction and Placentation

A subpopulation of NK cells are abundant in the uterus during the menstrual cycle and at the fetal-maternal interface, in particular, where fetal cells invade the uterine lining. It is thought that NK cells may exert their effect on placentation through release of cytokines. These cytokines may have effects on trophoblast differentiation and modulate the activity of maternal immune cells, for example macrophages and CD4 T cells, so that fetal cells are protected from immune attack thus preventing early failure of the pregnancy. Intrauterine NK cells may be triggered to release cytokines via interaction of the p70 NK cell receptor and p38.5 or p38.5 analogs on fetal cells.

Approximately 25% of infertility is due to immunologic causes, one of which may be defective NK cell triggering and release of protective cytokines. A patient with the above condition may be treated by injecting antibodies to p70 or p38.5 to cross-link and thus trigger the release of protective cytokines from intrauterine NK cells. The method of anti-p70 and anti-38.5 treatment has been described above except that antibodies will be raised to epitopes that are stimulatory rather than inhibitory.

Thus, while there have been described what are presently believed to be the preferred embodiments of the present invention, those skilled in the art will realize that other and further embodiments can be made without departing from the spirit of the invention, and it is intended to include all such further modifications and changes as come within the true scope of the claims set forth herein.

### Supplemental Enablement

The invention as claimed is enabled in accordance with the above specification and readily available references and starting materials. Nevertheless, Applicants have deposited with the American Type Culture Collection, Rockcille, Md., USA (ATCC) the cell lines listed below:
Nonactivated human Natural Killer Lymphocyte counter receptor nahNKCRp38.5 (f730), ATCC accession no. 98422, deposited May 1, 1997.
Nonactivated Human Natural Killer Lymphocyte Receptor nahNKRp70, ATCC accession no. 98424, deposited May 1, 1997.
Nonactivated human NK cell Library nahNKL, ATCC accession no. 98423, deposited May 1, 1997.

These deposits were made under the provisions of the Budapest Treaty on the International Recognition of the Deposit of Microorganisms for the Purposes of Patent Procedure and the regulations thereunder (Budapest Treaty). This assures the maintenance of a viable culture for 30 years from date of deposit. The organisms will be made available by ATCC under the terms of the Budapest Treaty, and subject to an agreement betweeen Applicants and ATCC which assures unrestricted availability upon issuance of the pertinent U.S. patent. Availability of the deposited strains is not to be construed as a license to practice the invention in contravention of the rights granted under the authority of any govenment in accordance with its patent laws.

## Claims

1. An isolated and purified cell surface protein p38.5 having a molecular weight of about 38.5 kD which binds Natural Killer (NK) cells and which is characteristically expressed by tumor cells susceptible to cell-mediated lysis by naive Natural Killer (NK) cells, wherein the protein comprises the amino acid sequence defined by SEQ ID NO:3.

2. A protein according to Claim 1, wherein the protein binds preferentially to NK cells in the presence of other types of lymphocytes.

3. A protein according to Claim 1, wherein the protein binds to p70 protein characteristically expressed on the surface of NK cells.

4. A protein according to Claim 1, wherein the protein is from a mammal.

5. A protein according to Claim 4, wherein the protein is from a human.

6. A nucleic acid encoding the cell surface protein p38.5 of claim 1.

7. A nucleic acid according to Claim 6, wherein the nucleic acid is DNA, RNA, or cDNA.

8. An antibody having specific binding affinity for an epitope of the cell surface protein p38.5 of claim 1.

9. An antibody according to Claim 8, wherein the antibody is selected from the group consisting of monoclonal antibodies, polyclonal antibodies, chimeric antibodies, humanized antibodies, Fv fragments, and Fab'₂ fragments.

10. An antibody according to Claim 8, which specifically binds to an epitope characteristic of the amino acid sequence defined by SEQ ID NO:3.

11. An in vitro diagnostic method for identifying tumor cells that are susceptible to natural killer (NK) cell-mediated lysis, comprising:
(a) providing a sample suspected of containing tumor cells obtained from an animal subject; and
(b) detecting expression of the p38.5 cell surface protein of claim 1 by the tumor cells; wherein detection of a measurable amount of the protein is indicative of susceptibility of said tumor cells being susceptible to NK cell-mediated lysis.

12. A method according to Claim 11, wherein the detecting comprises:
(i) contacting the tumor cells with an antibody having specific binding affinity for the p38.5 protein; and
(ii) measuring binding of the antibody to the tumor cells, wherein measurable binding of the antibody to the tumor cells is indicative of susceptibility of said tumor cells being susceptible to NK cell-mediated lysis.

13. A method according to Claim 11, wherein the detecting comprises:
(i) contacting said sample under hybridization conditions with an oligonucleotide probe that hybridizes specifically with a nucleic acid encoding the p38.5 protein; and
(ii) measuring hybridization of said oligonucleotide probe to said sample, wherein measurable hybridization of the oligonucleotide probe to the sample is indicative of the presence of tumor cells susceptible to NK cell-mediated lysis.

14. A method according to Claim 13, wherein the oligonucleotide probe binds to an mRNA encoding the p38.5 protein.

15. A method according to Claim 14, wherein said measuring comprises measuring amplification of nucleic acid encoding the p38.5 protein mediated through the oligonucleotide probe.

## Patentansprüche

1. Isoliertes und gereinigtes Zelloberflächenprotein p38.5 mit einer Molmasse von ungefähr 38,5 kD, das an natürliche Killerzellen (NK-Zellen) bindet und das charakteristisch von Tumorzellen exprimiert wird, die empfindlich gegenüber einer von naiven natürlichen Killerzellen (NK-Zellen) zellvermittelten Lyse sind, wobei das Protein die Aminosäurensequenz, die durch SEQ ID NO:3 definiert ist, umfasst.

2. Protein gemäß Anspruch 1, wobei das Protein in Gegenwart von anderen Lymphozytentypen bevorzugt an NK-Zellen bindet.

3. Protein gemäß Anspruch 1, wobei das Protein an p70-Protein bindet, das charakteristisch auf der Oberfläche von NK-Zellen exprimiert wird.

4. Protein gemäß Anspruch 1, wobei das Protein aus einem Säuger ist.

5. Protein gemäß Anspruch 4, wobei das Protein aus einem Menschen ist.

6. Nukleinsäure, die das Zelloberflächenprotein p38.5 von Anspruch 1 kodiert.

7. Nukleinsäure gemäß Anspruch 6, wobei die Nukleinsäure DNA, RNA oder cDNA ist.

8. Antikörper, der eine spezifische Bindungsaffinität für ein Epitop des Zelloberflächenproteins p38.5 von Anspruch 1 hat.

9. Antikörper gemäß Anspruch 8, wobei der Antikörper ausgewählt ist aus der Gruppe bestehend aus monoklonalen Antiköpern, polyklonalen Antikörpern, chimären Antikörpern, humanisierten Antiköpern, Fv-Fragmenten und Fab'₂-Fragmenten.

10. Antikörper gemäß Anspruch 8, der spezifisch an ein Epitop bindet, das die Aminosäurensequenz hat, die durch SEQ ID NO:3 definiert ist.

11. Diagnostisches *in vitro* Verfahren zum Identifizieren von Tumorzellen die empfindlich sind gegenüber einer von natürlichen Killerzellen vermittelten Lyse, umfassend:
(a) Bereitstellen einer Probe, von der man vermutet, dass sie Tumorzellen enthält, die von einem Tiersubjekt erhalten wurde; und
(b) Detektieren von Expression des p38.5-Zelloberflächenproteins von Anspruch 1 durch die Tumorzellen; wobei eine Detektion einer messbaren Menge des Proteins indikativ ist für Empfindlichkeit der Tumorzellen, die empfindlich gegenüber einer von NK-Zellen vermittelten Lyse sind.

12. Verfahren gemäß Anspruch 11, wobei das Detektieren umfasst:
(i) Kontaktieren der Tumorzellen mit einem Antikörper, der eine spezifische Bindungsaffinität für das p38.5 Protein hat; und
(ii) Messen des Bindens des Antikörpers an die Tumorzellen, wobei messbares Binden des Antikörpers an die Tumorzellen indikativ ist für die Empfindlichkeit der Tumorzellen, die empfindlich gegenüber einer von NK-Zellen vermittelten Lyse sind.

13. Verfahren gemäß Anspruch 11, wobei das Detektieren umfasst:
(i) Kontaktieren der Probe unter Hybridisierungsbedingungen mit einer Oligonukleotidsonde, die spezifisch mit einer Nukleinsäure, die das p38.5-Protein kodiert, hybridisiert; und
(ii) Messen von Hybridisierung der Oligonukleotidsonde an die Probe, wobei messbare Hybridisierung der Oligonukleotidprobe an die Probe indikativ ist für das Vorliegen von Tumorzellen, die empfindlich gegenüber einer von NK-Zellen vermittelten Lyse sind.

14. Verfahren gemäß Anspruch 13, wobei die Oligonukleotidsonde an mRNA bindet, die das p38.5-Protein kodiert.

15. Verfahren gemäß Anspruch 14, wobei das Messen Messen der durch die Oligonukleotidsonde vermittelten Amplifikation von p38.5-Protein kodierender Nukleinsäure, umfasst.

## Revendications

1. Protéine de surface cellulaire isolée et purifiée p38.5 ayant une masse moléculaire d'environ 38,5 kD qui se lie à des cellules tueuses naturelles (NK) et qui est caractéristiquement exprimée par des cellules tumorales prédisposées à subir une lyse cellulaire induite par des cellules tueuses naturelles (NK) naïves, ladite protéine comprenant la séquence d'acides aminés définie par la SEQ ID n° : 3.

2. Protéine selon la revendication 1, ladite protéine se liant de préférence à des cellules NK en présence d'autres types de lymphocytes.

3. Protéine selon la revendication 1, ladite protéine se liant à la protéine p70 caractéristiquement exprimée sur la surface de cellules NK.

4. Protéine selon la revendication 1, ladite protéine provenant d'un mammifère.

5. Protéine selon la revendication 4, ladite protéine provenant d'un humain.

6. Acide nucléique codant pour la protéine de surface cellulaire p38.5 de la revendication 1.

7. Acide nucléique selon la revendication 6, ledit acide nucléique étant un ADN, un ARN ou un ADNc.

8. Anticorps ayant une affinité de liaison spécifique pour un épitope de la protéine de surface cellulaire p38.5 de la revendication 1.

9. Anticorps selon la revendication 8, ledit anticorps étant sélectionné dans le groupe constitué d'anticorps monoclonaux, d'anticorps polyclonaux, d'anticorps chimères, d'anticorps humanisés, de fragments Fv et de fragments Fab'₂.

10. Anticorps selon la revendication 8, lequel se lie spécifiquement à un épitope caractéristique de la séquence d'acides aminés définie par la SEQ ID n° : 3.

11. Procédé de diagnostic *in vitro* servant à identifier des cellules tumorales qui sont prédisposées à subir une lyse induite par des cellules tueuses naturelles (NK), comprenant :
(a) de fournir un échantillon suspecté de contenir des cellules tumorales obtenu auprès d'un sujet animal ; et
(b) détecter l'expression de la protéine de surface cellulaire p38.5 de la revendication 1 par les cellules tumorales ; dans lequel la détection d'une quantité mesurable de la protéine est indicatrice de la prédisposition desdites cellules tumorales à être prédisposées à subir une lyse induite par des cellules NK.

12. Procédé selon la revendication 11, dans lequel la détection comprend :
(i) de mettre en contact les cellules tumorales avec un anticorps ayant une affinité de liaison spécifique pour la protéine p38.5 ; et
(ii) mesurer la liaison de l'anticorps aux cellules tumorales, dans lequel la liaison mesurable de l'anticorps aux cellules tumorales est indicatrice de la prédisposition desdites cellules tumorales à être prédisposées à subir une lyse induite par des cellules NK.

13. Procédé selon la revendication 11, dans lequel la détection comprend :
(i) de mettre en contact ledit échantillon dans des conditions d'hybridation avec une sonde oligonucléotide qui s'hybride spécifiquement avec un acide nucléique codant pour la protéine p38.5 ; et
(ii) mesurer l'hybridation de ladite sonde oligonucléotide audit échantillon, dans lequel l'hybridation mesurable de la sonde oligonucléotide à l'échantillon est indicatrice de la présence de cellules tumorales prédisposées à subir une lyse induite par des cellules NK.

14. Procédé selon la revendication 13, dans lequel la sonde oligonucléotide se lie à un ARNm codant pour la protéine p38.5.

15. Procédé selon la revendication 14, dans lequel ladite mesure comprend de mesurer l'amplification d'un acide nucléique codant pour la protéine p38.5 induite par la sonde oligonucléotide.
